# EUROPÄISCHE PATENTSCHRIFT

(11) **EP 4 400 143 B1**
(45) Veröffentlichungstag und Bekanntmachung des Hinweises auf die Patenterteilung: **14.01.2026**
(21) Anmeldenummer: 24178314.1
(22) Anmeldetag: 23.06.2020
(51) Int. Cl.: A61M 16/20, A61M 16/00, A61M 16/10

(54) **BEATMUNGSGERÄT ZUR STEUERUNG EINER GASQUELLE UND ZWEI DREHBEWEGTEN VENTILEN**
VENTILATOR FOR CONTROLLING A GAS SOURCE WITH TWO ROTARY VALVES
VENTILATEUR MÉCANIQUE POUR COMMANDER UNE SOURCE DE GAZ AVEC DEUX VALVES ROTATIVES

(30) Priorität: 02.07.2019 DE 102019004642
(43) Veröffentlichungstag der Anmeldung: 17.07.2024
(62) Teilanmeldung aus: 20740539.0
(73) Patentinhaber: Löwenstein Medical Technology S.A., 2557 Luxembourg (LU)
(72) Erfinder: Göbel, Christof, 22457 Hamburg (DE); Schattner, Jan, 25421 Pinneberg (DE)
(74) Vertreter: Löwenstein Medical IP

(56) Entgegenhaltungen:
- WO-A1-2013/068918
- WO-A1-2013/182944
- WO-A2-2011/073839
- WO-A2-2017/144963
- JP-A- H02 131 773
- US-A1- 2012 285 460

## Beschreibung

Beatmungsgeräte werden für die Therapie respiratorischer Störungen eingesetzt, dabei können die Beatmungsgeräte in der nicht-invasiven und invasiven Beatmung und in der Atemwegstherapie, sowohl innerklinisch als auch außerklinisch, verwendet werden. US 2012/285460 A1, JP H02 131773 A, WO 2011/073839 A2, WO 2013/068918 A1, WO 2013/182944 A1 und WO 2017/144963 A2 offenbaren Atemtherapiegeräte mit Druckerzeugern zur mechanischen Insufflation/Exsufflation oder zur Hochfrequenzbeatmung.

Die Aufgabe der Erfindung ist es ein verbessertes Beatmungsgerät anzugeben, welches für die Therapie respiratorischer Störungen in der nicht-invasiven und invasiven Beatmung und in der Atemwegstherapie und der Hustenunterstützung, sowohl innerklinisch als auch außerklinisch, verwendet werden kann.

Die Erfindung ist in den angehängten Ansprüchen definiert. Die Offenbarung betrifft ein Beatmungsgerät mit einer Gasquelle (beispielsweise einem Gebläse), zumindest einer Gasleitung und einer Patientenleitung und zumindest zwei Ventilen wobei jedes der Ventile zumindest mittelbar einen Anschluss zur Umgebungsluft aufweist und wobei die Ventile in der Gasleitung oder als Teil der Gasleitung angeordnet sind, wobei die Ventile zumindest zeitweise gasleitend mit der Gasquelle und der Patientenleitung oder der Umgebungsluft verbunden sind, wobei eines der Ventile ein Schaltventil ist, wobei das andere Ventil ein Oszillationsventil ist und in der Weise wirkt, dass durch schrittweises Öffnen und Schließen des Ventils der Strömungswiderstand in der Gasleitung zwischen Gebläse und Patient variiert werden kann, wodurch während der Insufflation und/oder der Exsufflation Oszillationen von Fluss und Druck bewirkt werden, wobei das Oszillationsventil zumindest eine Öffnung aufweist, die zu der Patientenleitung führt und eine weitere Öffnung die zu der Umgebungsluft führt und wobei das Ventil so ausgestaltet ist, dass es, abhängig von der Ventilstellung, die Öffnung, die zu der Patientenleitung führt oder die Öffnung, die zu der Umgebungsluft führt, für einen Atemgasfluss zumindest teilweise verschließt oder öffnet, wobei das Oszillationsventil als Drehventil mit einem Drehventilkörper, einem Motor und mit Öffnungen in mehreren Ebenen in einem Ventilgehäuse ausgebildet ist, wobei in einem Schaltzustand Pause eine Rotation des Drehventilkörpers die Öffnung mit einer Öffnung zur Umgebung freigibt und einen Gasfluss oder einen Druckabbau in die Umgebung ermöglicht und die Patientenleitung direkt, unter Umgehung des Schaltventils, mit der Umgebung verbunden ist.

Die Gasleitung kann offenbarungsgemäß zumindest Teilbereiche eines Gebläses und/oder zumindest eines Ventils oder Ventilkörpers umfassen.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des offenbarungsgemäßen Gegenstands lediglich das erste

Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Im Sinne der Offenbarung kann die Gasquelle eine Druckgasleitung, eine Druckgasquelle und/oder eine Ventilanordnung oder ein elektrisch betriebenes Gebläse sein.

Die Offenbarung betrifft ergänzend oder alternativ ein Beatmungsgerät, bei dem die Ventile zumindest zeitweise gasleitend mit der Gasquelle und der Patientenleitung oder der Umgebungsluft verbunden sind.

Die Offenbarung betrifft alternativ oder ergänzend auch ein Beatmungsgerät, bei dem die beiden Ventile in mindestens einer Schaltstellung und in mindestens einem Teil der Gasleitung strömungstechnisch in Reihe geschaltet sind.

Die Offenbarung betrifft alternativ oder ergänzend zudem ein Beatmungsgerät welches sich dadurch auszeichnet, dass eines der Ventile (Schaltventil) in der Weise schaltend wirkt, dass in einer ersten Schaltstellung eine Insufflation des Patienten und in einer zweiten Schaltstellung eine Exsufflation des Patienten bereitgestellt werden.

Eine weitere vorteilhafte Ausgestaltung der Offenbarung sieht vor, dass zumindest eine Steuereinheit eingerichtet und ausgebildet ist, die Gasquelle zu steuern und/oder das Schaltventil zu steuern und/oder das Oszillationsventil zu steuern.

Die Offenbarung sieht auch vor, dass die Steuereinheit eingerichtet und ausgebildet ist, das Gebläse zu steuern und/oder das Schaltventil zu steuern und/oder das Oszillationsventil zu steuern, wobei für die Insufflation durch das Gebläse für eine definierte Zeit ein entsprechend hoher Druck vorgegeben wird und dann durch das Schaltventil und/oder das Gebläse auf die Exsufflation umgeschaltet wird wozu der Druck innerhalb einer definierten Zeitspanne auf ein entsprechend negatives Niveau abgesenkt und für eine bestimmte Dauer gehalten wird, wobei anschließend eine erneute Anhebung des Drucks auf das gewünschte Niveau für die Insufflation erfolgt, wobei die Umschaltung insbesondere durch das Schaltventil und/oder das Gebläse erfolgt.

Die Offenbarung sieht ergänzend auch vor, dass die Steuereinheit eingerichtet und ausgebildet ist, das Gebläse zu steuern und/oder das Schaltventil zu steuern und/oder das Oszillationsventil zu steuern, wobei für die Insufflation durch das Gebläse für eine definierte Zeit ein entsprechend hoher Druck vorgegeben wird und dann durch das Schaltventil und/oder das Gebläse auf die Exsufflation umgeschaltet wird wozu der Druck innerhalb einer definierten Zeitspanne auf ein entsprechend negatives Niveau abgesenkt und für eine bestimmte Dauer gehalten wird, wobei anschließend durch das Gebläse eine erneute Anhebung des Drucks auf das gewünschte Niveau für die Pause erfolgt, wobei der Druck während der Pause einen leichten Überdruck aufweist, welcher insbesondere zwischen 2 und 15 mbar liegt.

Die Offenbarung sieht auch vor, dass die Steuereinheit eingerichtet und ausgebildet ist, das Gebläse zu steuern und/oder das Schaltventil zu steuern und/oder das Oszillationsventil zu steuern, wobei der Abfall des Drucks bei dem Übergang von Insufflation zu Exsufflation durch ein entsprechend Umschalten des Schaltventils erfolgt, sodass ein Gasfluss von der Patientenleitung über die Saugseite des Gebläses zur Umgebung 1 erfolgt.

Die Offenbarung sieht auch vor, dass die Steuereinheit eingerichtet und ausgebildet ist, das Gebläse zu steuern und/oder das Schaltventil zu steuern und/oder das Oszillationsventil zu steuern, wobei der Anstieg des Drucks von der Exsufflation zur nächsten Insufflation oder nach einer Pause hin zur folgenden Insufflation erfolgt vorzugsweise weniger zügig bzw. über einen längeren Zeitraum, wobei der Druckanstieg neben der Änderung der Ventilstellung des Schaltventils auch durch ein entsprechendes Hochfahren des Gebläses erfolgt.

Die Offenbarung sieht auch vor, dass die Steuereinheit eingerichtet und ausgebildet ist, das Gebläse zu steuern und/oder das Schaltventil zu steuern und/oder das Oszillationsventil zu steuern, wobei auf dem Druckniveau der Inspiration 30 oder dem der Exspiration oder in der Umschaltphase zwischen Inspiration 30 und Exspiration oder während der Pause eine definierte Oszillation erfolgt, wobei das Oszillationsventil 3 dabei in der Weise wirkt, dass durch schrittweises Öffnen und Schließen dieses Ventils der Strömungswiderstand in der Gasleitung zwischen Gebläse und Patient in der Art variiert werden kann, dass Oszillationen von Fluss und/oder Druck bewirkt werden.

Die Offenbarung sieht auch vor, dass die Steuereinheit eingerichtet und ausgebildet ist, das Gebläse zu steuern und/oder das Schaltventil zu steuern und/oder das Oszillationsventil zu steuern, wobei für ein Hustenmanöver das Schaltventil in die Ventilstellung für die Insufflation gebracht wird und dann die Drehzahl des Gebläses erhöht wird, wodurch sich entsprechend der Druck erhöht, wobei nach Erreichen des für die Insufflation erforderlichen Drucks die Drehzahl gehalten wird und für den Wechsel zu der Exsufflation das Schaltventil in die Ventilstellung für die Exsufflation geschaltet wird, wodurch der Druck über einen kurzen Zeitraum entsprechend abfällt und so der für die Exsufflation notwendige negative Druck erreicht wird und der Druck und/oder die Drehzahl des Gebläses für die Exsufflation für eine vorbestimmte Zeit gehalten werden.

Eine ergänzende Ausgestaltung der Offenbarung sieht vor, dass zumindest eines der Ventile als drehbar gelagertes Ventil (Drehventil) ausgeführt ist.

Eine noch weitere vorteilhafte Ausgestaltung der Offenbarung sieht vor, dass zumindest eines der Ventile als Axialventil ausgeführt ist, wobei für ein Axialventil eine axiale oder lineare Bewegungsführung für einen Öffnungs- oder Schließvorgang charakteristisch ist.

Eine noch ergänzende vorteilhafte Ausgestaltung der Offenbarung sieht vor, dass das Schaltventil einen elektrisch angetriebenen Motor mit einem Stator und einen drehfest am Rotor befestigten Ventilkörper aufweist, wobei der Ventilkörper um eine Drehachse drehbar gelagert ist und aufgrund der Drehachse eine Radial- und eine Axialrichtung des Ventilkörpers definiert sind.

Eine noch weitere oder ergänzende vorteilhafte Ausgestaltung der Offenbarung sieht vor, dass das Oszillationsventil einen elektrisch angetriebenen Motor mit einem Stator und einen drehfest am Rotor befestigten Ventilkörper aufweist wobei der Ventilkörper um eine Drehachse drehbar gelagert ist und aufgrund der Drehachse eine Radial- und eine Axialrichtung des Ventilkörpers definiert sind.

Eine alternative oder ergänzende vorteilhafte Ausgestaltung der Offenbarung sieht vor, dass das Oszillationsventil als drehbar gelagertes Ventil ausgeführt ist und der Ventilkörper zumindest eine Öffnung in Radialrichtung und zumindest eine Öffnung in Axialrichtung aufweist.

Eine Öffnung ist im Sinne der Offenbarung ein Durchbruch oder eine Aussparung im Ventil oder ein Teilbereich, der einen Gasfluss zumindest zeitweise ermöglicht. Die Öffnung kann dabei auch zwischen zwei Bauteilen entstehen, die funktional so zusammenwirken, dass sie zumindest zeitweise eine Öffnung für eine Gasströmung freigeben. Eine Öffnung ist erfindungsgemäß dann ausgebildet, wenn zumindest zeitweise eine Gasströmung durch die Öffnung oder entlang der Öffnung möglich ist.

Eine weitere alternative oder ergänzende vorteilhafte Ausgestaltung der Offenbarung sieht vor, dass die Öffnung in Axialrichtung zum Anschluss, der zur Umgebungsluft führt, weist.

Eine vorteilhafte Ausgestaltung der Offenbarung sieht vor, dass der Ventilkörper einen zentralen Bereich aufweist, der sich zylinderförmig in Axialrichtung um die Aufnahme für die Motorwelle, erstreckt und der Ventilkörper zudem eine Deckscheibe aufweist, die sich ausgehend von dem oberen Ende des zentralen Bereiches in radialer Richtung erstreckt und an der radialen Außenkante oder nahe der Außenkante der Deckscheibe ein Wandbereich, der sich in axialer Richtung oder in einem rechten Winkel von der Deckscheibe ausgehend und im Wesentlichen parallel zu dem zentralen Bereich erstreckt, wobei sich zwischen dem Wandbereich und dem zentralen Bereich ein Kanal der gasführend ist erstreckt und die Öffnungen über den Kanal gasführend verbunden sind.

Gemäß einer anderen vorteilhaften Weiterbildung weist die Deckscheibe eine Öffnung auf, die in einer Drehposition des Ventils mit dem Anschluss zur Umgebung gasführend verbunden ist.

Gemäß einer weiteren vorteilhaften Weiterbildung sind die Form der Öffnung und/oder die Form des Anschlusses so ausgeführt, dass die Überlappung zwischen der Öffnung und dem Anschluss, bei Rotation des Ventils je nach Drehrichtung linear anwächst oder abschwillt.

Eine weitere vorteilhafte Ausgestaltung der Offenbarung sieht vor, dass das Gebläse, welches eine Saugseite und eine Druckseite aufweist mit zumindest einer Steuereinheit, wobei die Druckseite gasleitend mit einem Schaltventil verbunden und einem Oszillationsventil verbunden ist, wobei die Saugseite mit dem Schaltventil und dem Oszillationsventil verbunden ist wobei das Schaltventil einen Anschluss zur Saugseite und einen Anschluss zur Druckseite und einen Anschluss zur Umgebungsluft aufweist und wobei das Oszillationsventil einen Anschluss zur Patientenleitung und einen Anschluss zur Druckseite und einen Anschluss zur Umgebungsluft aufweist.

Die Offenbarung sieht in einer noch ergänzenden vorteilhaften Ausgestaltung vor, dass das Schaltventil und das Oszillationsventil in Reihe zwischen dem Gebläse und der Patientenleitung angeordnet sind, wobei der Anschluss zur Patientenleitung des Schaltventils zu dem Anschluss zur Druckseite des Oszillationsventils führt und das Schaltventil insofern mittelbar mit der Patientenleitung verbunden ist und das Oszillationsventil mittelbar mit der Druckseite verbunden ist.

Die Offenbarung sieht in einer noch weiteren, ergänzenden und vorteilhaften Ausgestaltung vor, dass die Steuereinheit eingerichtet und ausgebildet ist, das Gebläse zu steuern und/oder das Schaltventil zu steuern und/oder das Oszillationsventil zu steuern.

Die Offenbarung sieht in einer weiteren, ergänzenden und vorteilhaften Ausgestaltung vor, dass das Schaltventil und das Oszillationsventil eine gemeinsame Steuereinheit oder separate Steuereinheiten aufweisen, wobei alternativ oder ergänzend diese Steuereinheiten mit der Steuereinheit kommunizieren, die das Gebläse steuert. Es ist auch vorgesehen, dass lediglich eine Steuereinheit das Gebläse, das Schaltventil und das Oszillationsventil steuert. Das Gebläse, das Schaltventil und das Oszillationsventil können auch je eine Steuereinheit aufweisen, die alle durch eine zentrale Steuereinheit koordiniert und gesteuert werden.

Die Offenbarung sieht in einer ergänzenden vorteilhaften Ausgestaltung vor, dass das Schaltventil Schaltmittel aufweist, welche als Motor, als Anschläge und als Ventilkörper in einem Ventilgehäuse ausgebildet sind.

Die Offenbarung sieht in einer anderen vorteilhaften Ausgestaltung vor, dass die Anschläge in dem Ventilgehäuse ausgebildet sind.

Die Offenbarung sieht in einer weiteren vorteilhaften Ausgestaltung vor, dass das der Motor den Ventilkörper zwischen zumindest drei Schaltzuständen rotiert, wobei die maximale Rotation zwischen den Anschlägen maximal 150° ist, bevorzugt maximal 120°, besonders bevorzugt maximal 100° ist.

Die Offenbarung sieht in einer vorteilhaften Ausgestaltung vor, dass die Schaltzustände für das Schaltventil Einatmung, Ausatmung und Pause sind, wobei für die Einatmung die Stellung des Ventilkörpers einen Gasfluss von der Umgebung zur Saugseite des Gebläses freigibt und gleichzeitig einen Gasfluss von der Druckseite über den Anschluss zu dem Anschluss des zweiten Ventils ermöglicht.

Nach einer weiteren vorteilhaften Ausgestaltung sieht die Offenbarung vor, dass die Schaltzustände für das Schaltventil Einatmung, Ausatmung und Pause sind, wobei für die Ausatmung die Stellung des Ventilkörpers einen Gasfluss von dem Anschluss des zweiten Ventils über den Anschluss zur Saugseite des Gebläses freigibt und einen Gasfluss von der Druckseite zu der Umgebung.

Nach einer ergänzenden vorteilhaften Ausgestaltung sieht die Offenbarung vor, dass die Schaltzustände für das Schaltventil Einatmung, Ausatmung und Pause sind, wobei für die Pause die Stellung des Ventilkörpers einen Gasfluss von der Saugseite des Gebläses zu der Druckseite freigibt, wodurch der Gasfluss in mindestens einer der beiden Richtungen von der Umgebung hin zum Patienten oder vom Patienten hin zur Umgebung im Wesentlichen unterbunden wird.

Nach einer noch anderen vorteilhaften Ausgestaltung sieht die Offenbarung vor, dass Motor ein Schrittmotor ist.

Nach einer weiteren vorteilhaften Ausgestaltung sieht die Offenbarung vor, dass die Anschläge an dem Ventilgehäuse ausgebildet sind.

Nach einer alternativen vorteilhaften Ausgestaltung sieht die Offenbarung vor, dass der Drehventilkörper Öffnungen aufweist, die in radiale Richtung und axiale Richtung weisen.

Nach einer ergänzenden vorteilhaften Ausgestaltung sieht die Offenbarung vor, dass die Öffnung zur Umgebungsluft axial in der Deckscheibe des Drehventilkörpers angeordnet ist und eine Rotation des Drehventilkörpers die Öffnung in Überdeckung mit dem Anschluss 36 zur Umgebungsluft bringt.

Nach einer weiteren vorteilhaften Ausgestaltung sieht die Offenbarung vor, dass die Öffnungen radial im Drehventilkörper angeordnet sind und in der entsprechenden Schaltstellung des Drehventilkörpers, einen Gasfluss von der Druckseite über den Anschluss des Schaltventils zu dem Anschluss des zweiten Ventils und weiter über den Anschluss zu der Patientenleitung ermöglichen.

Eine ergänzende vorteilhafte Ausgestaltung der Offenbarung sieht vor, dass in einem Schaltzustand Oszillation eine oszillierende Bewegung des Ventilkörpers des Oszillationsventils mit einer bestimmten Frequenz vorgesehen ist, die die Öffnung mit der Umgebung freigibt und wieder verschließt, was einen zeitweisen, frequenzabhängigen Gasfluss oder Druckabbau in die Umgebung bedingt, wodurch der Gasfluss in mindestens einer der Richtungen hin zum oder weg vom Patienten mit Druck- und Flussoszillationen überlagert wird.

Eine alternative vorteilhafte Ausgestaltung der Offenbarung sieht vor, dass die oszillierende Bewegung eine rotatorische Bewegung des drehbar gelagerten Ventilkörpers eines Oszillationsventils ist.

Eine andere vorteilhafte Ausgestaltung der Offenbarung sieht vor, dass die oszillierende Bewegung eine axiale Bewegung eines axial gelagerten Ventilkörpers eines Oszillationsventils ist.

Eine weitere vorteilhafte Ausgestaltung der Offenbarung sieht vor, dass die Anschläge hart ausgeführt sind.

Für die Erkennung der Position der Ventile können zusätzlich Drehwinkelgeber bzw. Lichtschranken genutzt werden.

Bezüglich beatmungsgerätbezogener Begriffsdefinitionen sowie der Wirkungen und Vorteile beatmungsgerätgemäßer Merkmale wird vollumfänglich auf die Erläuterungen sinngemäßer Definitionen, Wirkungen und Vorteile des gesamten Textes verwiesen. Offenbarungen hierin bezüglich des offenbarungsgemäßen Beatmungsgerätes sollen in sinngemäßer Weise auch zur Definition des offenbarungsgemäßen Verfahrens herangezogen werden können, sofern dies hierin nicht ausdrücklich ausgeschlossen ist. Insofern wird auf eine Wiederholung von Erläuterungen sinngemäß gleicher Merkmale im Verlaufe des gesamten Textes, deren Wirkungen und Vorteile bezüglich des hierin offenbarten offenbarungsgemäßen Verfahrens sowie des hierin offenbarten offenbarungsgemäßen Beatmungsgeräts, zugunsten einer kompakteren Beschreibung weitgehend verzichtet.

Die Offenbarung bietet unter anderem folgende Vorteile:
Durch die Öffnung im Oszillationsventil kann eine schnelle Entlastung des Patienten stattfinden und er muss nicht durch das Gebläse atmen.

Durch eine Öffnung zur Umgebung, mit der das Oszillationsventil kommuniziert, sind größere Druckamplituden möglich, da Luft aus dem System entweichen und der Druck wirksam und schnell abgebaut werden kann.

Das Oszillationsventil muss um maximal 90° rotieren um die Oszillation zu ermöglichen; die Bewegungen für den Motor sind klein.

Asymmetrische Muster der Druck- oder Fluss-oszillation lassen sich durch hin- und herbewegen des Oszillationsventils schnell erzeugen.

Die Aufteilung der beiden Funktionen in Schalten der Strömungsrichtung und Generieren von Oszillation auf dem Fluss ist durch getrennte Ventile günstiger möglich. Einzelventile sind einfacher und schneller zu fertigen und ihre jeweilige Funktion ist leichter technisch zu realisieren und zu optimieren.

Einzelventile können beispielsweise mit geringerer Massenträgheit gefertigt werden als Ventile, die beide Funktionen in einem erfüllen sollen, diese würden typischerweise größer realisiert werden müssen. Daraus resultieren Dynamikvorteile während der Schaltvorgängge und/oder Oszillationen. Toleranzbetrachtungen sind weniger kritisch, weil die Ventilkörper weniger komplex sind.

Die Dichtigkeit ist leichter herzustellen, weil für jedes Ventil weniger mögliche Schaltstellungen vorhanden sind. Unter anderem ist bei geringen möglichen Toleranzen angedacht, auf zusätzliche Bauteile wie nachgiebige Dichtungen verzichten zu können.

Eine klare Zuweisung der verschiedenen Funktionen Schalten für Hustenmanöver und Oszillation während der verschiedenen Phasen eines Hustenmanövers kann auf die zwei Ventile verteilt werden.

Die Ansteuerung der Ventile kann separat entwickelt und optimiert werden und muss nicht für ein Ventil mit komplexerer Gesamtfunktion erfolgen.

Als eine Antriebsart für die Ventile sind offenbarungsgemäß Linearantriebe angedacht, wobei Schrittmotoren für rotierend betriebene Ventile als günstige Massenware beispielsweise Kostenvorteile mit sich bringen.

Das Fahren in Anschläge mit den Ventilen ermöglicht eine Erkennung der Ventilstellung ohne zusätzliche Sensorik. Dies kann beispielsweise während bestimmter Einmessmanöver am Ende von Servicevorgängen oder nach jedem Gerätestart erfolgen.

Je nach Wahl der Öffnung des Oszillationsventils besteht die Möglichkeit, der Strömung hin zum oder weg vom Patienten Oszillationen von Fluss und Druck mit kleineren oder größeren Amplituden zu überlagern.

Ein genaues Einstellen der Oszillation, kann je nach Form der Öffnung erfolgen. Der Anwender kann Oszillationen mit verschieden großen Amplituden einstellen, die durch verschiedene Drehwinkel-Bereiche des Oszillationsventils während der Oszillation realisiert werden.

Die Oszillation ist in Geräten zur maschinellen Beatmung und in Geräten zur Hustenassistenz nutzbar. Offenbarungsgemäß werden alle diese Geräte und auch weitere Geräte zur Atemtherapie oder Beatmung vereinfachend als Beatmungsgeräte bezeichnet.

Eine vorteilhafte Ausgestaltung der Offenbarung sieht vor, dass das Beatmungsgerät wenigstens eine Datenübertragungsschnittstelle aufweist, die ausgebildet und eingerichtet ist, den in der Speichereinheit gespeicherten Inhalt des Häufigkeitszählers bezüglich Hustenereignissen an einen Datenempfänger bzw. eine Datenempfangseinheit zu übertragen. Die Datenübertragung kann hierbei drahtgebunden oder drahtlos erfolgen. Datenübertragungsschnittstellen können beispielsweise ein elektronischer Datenübertragungsbus, eine Netzwerkschnittstelle (z. B. LAN) ein Datenübertragungsmodem, eine USB-Schnittstelle, eine Funkübertragungsschnittstelle wie z. B. Infrarot, Bluetooth, WIFI, GSM/LTE und dergleichen oder auch ein Wechselspeichermedium wie z. B. eine Speicherkarte (Flash), ein USB-Stick/-Festplatte und dergleichen sein. Der Datenempfänger bzw. die Datenempfangseinheit kann beispielsweise eine geräteexterne (entfernte) Datenverarbeitungseinrichtung mit einer Auswerteeinheit (z. B. Auswertesoftware) sein, ein Expertenanwender (z. B. Arzt), eine Anzeigeeinrichtung (z. B. Display, Monitor) und dergleichen. Die Datenempfangseinheit kann somit die Befundung des Analyseergebnisses zu einem späteren Zeitpunkt ermöglichen, insbesondere in dem Fall, in dem das Beatmungsgerät keine geräteinterne Auswerteeinheit aufweist. Das Beatmungsgerät kann jedoch alternativ oder zusätzlich eine interne Auswerteeinheit aufweisen.

Nach einer weiteren vorteilhaften Ausgestaltung der Offenbarung weist das Beatmungsgerät eine weitere Sensoreinheit auf, die ausgebildet ist, eine Drehzahl eines Beatmungsgebläses und/oder einen Leckageverlust des Atemgases bei der Beatmung der Person und/oder einen Atemzugtyp wie spontane Atmung oder mandatorische Atmung zu erfassen und dem Atemzuganalysator zuzuführen.

Gemäß einer anderen vorteilhaften Weiterbildung weist das Beatmungsgerät eine Auswerteeinheit auf, die ausgebildet und eingerichtet ist, das Analyseergebnis des Analysators hinsichtlich hustenähnlicher Komplikationszustände der beatmeten Person auszuwerten und in der Speichereinheit zu speichern und/oder auf einer Anzeigeeinrichtung anzuzeigen und/oder an eine geräteexterne Datenempfangseinheit zu übertragen. Die Datenübertragung kann hierzu insbesondere über die vorgenannte Datenübertragungsschnittstelle erfolgen. Als Datenempfangseinheit, sind insbesondere eine Überwachungseinheit ein Monitor in einem Krankenhaus oder Pflegeheim und bei häuslicher Beatmung ein Telemonitoring-Server zu verstehen. Falls die Häufigkeit und/oder der Schweregrad einer festgestellten Komplikation einen festgelegten Grenzwert überschreitet, kann das Beatmungsgerät ebenfalls ausgebildet sein, einen Alarm auszugeben, beispielsweise auf der Anzeigeeinrichtung und/oder einen Alarm an die Datenempfangseinheit zu senden.

Es sei angemerkt, dass bezüglich verfahrensbezogener Begriffsdefinitionen sowie der Wirkungen und Vorteile verfahrensgemäßer Merkmale vollumfänglich auf die vorstehenden Erläuterungen sinngemäßer Definitionen, Wirkungen und Vorteile bezüglich der offenbarungsgemäßen

Vorrichtung(en) verwiesen wird. Dementsprechend können Offenbarungen hierin bezüglich der offenbarungsgemäßen Vorrichtung(en) in sinngemäßer

Weise auch zur Definition des offenbarungsgemäßen Verfahrens herangezogen werden und

Offenbarungen hierin bezüglich des offenbarungsgemäßen Verfahrens können in sinngemäßer Weise zur Definition der erfindungsgemäßen Vorrichtung(en) herangezogen werden. Auf eine Wiederholung von Erläuterungen sinngemäß gleicher Merkmale, deren Wirkungen und Vorteile wird somit weitgehend verzichtet.

Weitere Merkmale und Vorteile der Offenbarung ergeben sich aus der folgenden Beschreibung, nicht einschränkend zu verstehender Ausführungsbeispiele der Erfindung, die im Folgenden unter Bezugnahme auf die Zeichnungen näher erläutert wird.

Die Begriffe Einatmung, Insufflation, Inspiration werden in dieser Anmeldung als Synonyme verwendet, ebenso wir die entsprechenden Begriffe für die Ausatmung Exsufflation, Exspiration.

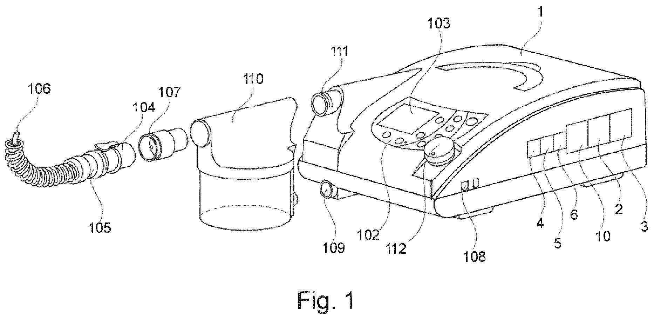

Die Figur 1 zeigt ein offenbarungsgemäßes Beatmungsgerät 1. Das Beatmungsgerät 1 kann als Beatmungsgerät für Heim- oder Klinikanwendungen und/oder als Hustentherapiegerät und/oder als kombiniertes Beatmungs- und Hustentherapiegerät ausgebildet sein.

Das Beatmungsgerät 1 ist mit einer Gebläseeinrichtung 10 bzw. einem Lüfter ausgestattet, um eine Atemluftströmung zur Beatmung des Patienten zu erzeugen. Das Beatmungsgerät 1 kann zusätzlich zumindest ein Ventil 2, 3 umfassen. Das Beatmungsgerät 1 umfasst beispielsweise zumindest ein Gebläse 10 und ergänzend zumindest ein Ventil 2, 3 mit welchen eine Atemluftströmung für eine Einatmung und eine Atemluftströmung für eine Ausatmung erzeugt oder moduliert wird.

Das Beatmungsgerät 1 umfasst alternativ beispielsweise zwei Gebläse und zumindest ein Ventil mit welchen eine Atemluftströmung für eine Einatmung und eine Atemluftströmung für eine Ausatmung erzeugt oder moduliert wird. Das Gebläse und/oder das Ventil sind optional zusätzlich ausgebildet und eingerichtet, eine Atemluftströmung für eine Einatmung und eine Atemluftströmung für eine Ausatmung mit einer definierten Oszillation zu überlagern.

Das Gebläse und/oder das Ventil sind optional zusätzlich ausgebildet und eingerichtet, die Atemluftströmung für eine Ausatmung durch einen Unterdruck zu erzeugen oder zumindest zu unterstützen. Mit Unterdruck ist hierbei gemeint, dass der durch das Beatmungsgerät erzeugte Druck unterhalb des atmosphärischen Druckniveaus liegt.

Über eine Anschlusseinrichtung 111, 109 kann ein Atemschlauchsystem 104 - 107 angeschlossen werden. Die Anschlusseinrichtung ist Teil der Patientenleitung 14. Zudem umfasst das Beatmungsgerät hier beispielsweise eine Verneblereinrichtung 110, um zum Beispiel Medikamente in der Atemluft zu vernebeln oder einen Anfeuchter 110.

Das Beatmungsgerät dient auch zur gezielten Unterstützung einer Sekretabfuhr aus den Atemwegen eines Patienten und umfasst zumindest ein Gebläse 10 und ein Ventil 2,3.

Das Schlauchsystem umfasst ein Patienteninterface, dass beispielsweise eine Maske oder ein Mundstück sein kann. Darüber hinaus wird optional ein Patientenfilter oder Bakterienfilter verwendet, der das Schlauchsystem und/oder Beatmungsgerät während der Husten- oder Rückatemphasen vor einer Kontamination mit Keimen schützt. Das Schlauchsystem kann darüber hinaus optional mit einem Ausatemsystem oder einem schaltbaren Patientenventil ausgestattet sein, wobei sich in der einfachsten Ausführungsform neben dem Patienteninterface und optionalen Filter kein weiteres Zubehör im Schlauchsystem befindet. Über ein Ausatemsystem oder Patientenventil kann beispielsweise kontinuierlich oder schaltend CO2- reiche Ausatemluft abführbar sein. Das Abführen von Ausatemluft kann aber auch gezielt an Atemphasen bzw. Hustenphasen angepasst werden. Ausatemluft kann auch über das zumindest eine Ventil über zumindest einen Anschluss 26, 36 zur Umgebungsluft abgeleitet werden.

Das Beatmungsgerät 1 weist zudem eine Anzeigeeinrichtung 103 und eine Bedieneinrichtung 102, 112 zur Vornahme von Eingaben und Einstellungen auf. Die Bedieneinrichtung 102 kann als Touchscreen ausgebildet sein und/oder als mechanisches Bedienelement 112.

Zur Ansteuerung von zumindest Gebläse und Ventil ist jeweils eine oder mehrere Steuereinrichtungen 4 vorgesehen. Gebläse und Ventil oder Anzeige können auch teilweise eigene bzw. separate Steuereinrichtungen aufweisen. In der Steuereinrichtung sind vorzugsweise Vorgaben zur Ansteuerung von Gebläse und Ventil hinterlegt bzw. gespeichert. Diese Vorgaben können insbesondere durch den Benutzer bzw. einen Betreuer wenigstens teilweise angepasst werden. Die Steuereinrichtung 4 umfasst beispielsweise wenigstens einen Controller und/oder andere Steuerkomponenten.

Das Beatmungsgerät 1 weist zumindest eine Schnittstelle 108 auf. Über die Schnittstelle können beispielsweise mehrere Beatmungsgeräte oder ein Beatmungsgerät mit einem Patientenmonitor oder einem Krankenhausinformationssystem drahtlos und/oder drahtgebunden kommunizieren. Die Schnittstelle 108 ist beispielsweise mit der Steuereinrichtung 4 wirkverbunden.

Eine vorteilhafte Ausgestaltung der Offenbarung sieht vor, dass das Beatmungsgerät wenigstens eine Schnittstelle aufweist, die ausgebildet und eingerichtet ist, den in der Speichereinheit gespeicherten Inhalt des Häufigkeitszählers bezüglich Hustenereignissen an einen Datenempfänger bzw. eine Datenempfangseinheit zu übertragen. Die Datenübertragung kann hierbei drahtgebunden oder drahtlos erfolgen. Datenübertragungsschnittstellen können beispielsweise ein elektronischer Datenübertragungsbus, eine Netzwerkschnittstelle (z. B. LAN) ein Datenübertragungsmodem, eine USB-Schnittstelle, eine Funkübertragungsschnittstelle wie z. B. Infrarot, Bluetooth, WIFI, GSM/LTE und dergleichen oder auch ein Wechselspeichermedium wie z. B. eine Speicherkarte (Flash), ein USB-Stick/- Festplatte und dergleichen sein. Der Datenempfänger bzw. die Datenempfangseinheit kann beispielsweise eine geräteexterne (entfernte) Datenverarbeitungseinrichtung mit einer Auswerteeinheit (z. B. Auswertesoftware) sein, ein Expertenanwender (z. B. Arzt), eine Anzeigeeinrichtung (z. B. Display, Monitor) und dergleichen. Die Datenempfangseinheit kann somit die Befundung des Analyseergebnisses zu einem späteren Zeitpunkt ermöglichen, insbesondere in dem Fall, in dem das Beatmungsgerät keine geräteinterne Auswerteeinheit aufweist. Das Beatmungsgerät kann jedoch alternativ oder zusätzlich eine interne Auswerteeinheit aufweisen.

Das Beatmungsgerät 1 bietet hier alternativ die Möglichkeit, über die Schnittstelle 108 eine oder mehrere externe Gerätekomponenten (Anfeuchter, Vernebler, Sauerstoffmischer ...) zu koppeln und somit das Beatmungsgerät 1 funktionell zu erweitern oder zu ersetzen.

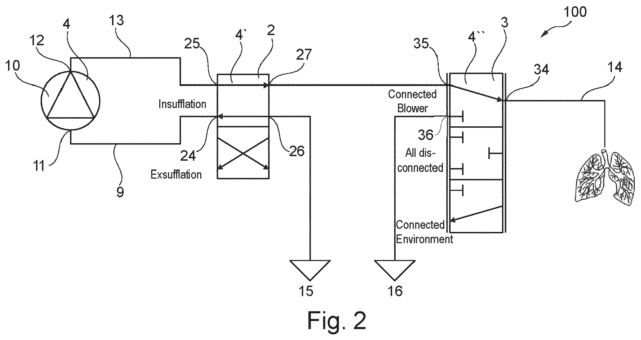

Figur 2 zeigt: Das offenbarungsgemäße Beatmungsgerät 100 weist zumindest ein Gebläse 10 auf, mit einer Saugseite 11 und einer Druckseite 12, und zumindest eine Steuereinheit 4 und eine Gasleitung 13 welche die Druckseite 12 gasleitend mit einer Patientenleitung 14 verbindet. Die Gasleitung 13 ist mit einem Schaltventil 2 und einem Oszillationsventil 3 verbunden. Eine Sauggasleitung 9 verbindet die Saugseite 11 des Gebläses mit dem Schaltventil 2.

Das Schaltventil 2 weist einen Anschluss 24 zur Saugseite 11 und einen Anschluss 25 zur Druckseite 12, einen Anschluss 26 zur Umgebungsluft 15 und einen Anschluss 27 zur Patientenleitung 14 auf. Das Oszillationsventil 3 weist einen Anschluss 34 zur Patientenleitung 14 und einen Anschluss 35, zu dem Schaltventil 2 und einen Anschluss 36 zur Umgebungsluft 16 auf.

Das Schaltventil 2 und das Oszillationsventil 3 sind zwischen dem Gebläse 10 und der Patientenleitung 14 in Reihe angeordnet. Beispielsweise sind beide Ventile in einem gemeinsamen Ventilblock angeordnet. Der Anschluss 27 des Schaltventils 2 ist über die Gasleitung 13 mit dem Anschluss 35 des Oszillationsventils 3 verbunden und der Anschluss 34 ist mit der Patientenleitung 14 verbunden. Das Gebläse ist insofern über das Schaltventil 2 und das Oszillationsventil 3 mittelbar mit der Patientenleitung 14 verbunden. Das Beatmungsgerät ist eingerichtet und ausgebildet eine, zumindest zeitweilige, Gasströmung zwischen der Umgebung 15 und der Patientenleitung 14 herzustellen, wobei die Gasströmung zumindest abschnittsweise in beiden Richtungen erfolgen kann.

Die zumindest eine Steuereinheit 4 ist eingerichtet und ausgebildet, das Gebläse 10 zu steuern und/oder das Schaltventil 2 zu steuern und/oder das Oszillationsventil 3 zu steuern.

Die zumindest eine Steuereinheit 4 kann benachbart zu den Ventilen und dem Gebläse 10 angeordnet sein oder entfernt von diesen und per Datenleitung mit diesen verbunden sein. Die zumindest eine Steuereinheit 4 steuert die Schaltzustände für das Schaltventil 2 das Oszillationsventil 3 nämlich Einatmung, Ausatmung und Pause.

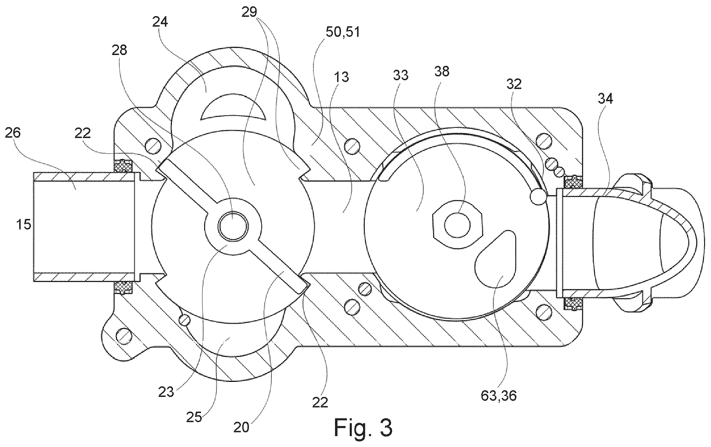

Figur 3 zeigt: Das Schaltventil 2 und das Oszillationsventil 3 sind in dem Ventilblock 50, hier dargestellt in der Oberhälfte, zwischen der Umgebung 15 und der Patientenleitung 14 in Reihe angeordnet. Die Gasleitung 13 verbindet Schaltventil 2 und das Oszillationsventil 3 und ist in dem Ventilblock realisiert. Das Schaltventil 2 weist einen Anschluss 24 zur Saugseite 11 und einen Anschluss 25 zur Druckseite 12 des Gebläses auf. Ein weiterer Anschluss führt zur Umgebungsluft 15 und ein Anschluss 27 über die Gasleitung 13 zum Oszillationsventil 3 und zur Patientenleitung 14. Das Schaltventil 2 ist in dem Ventilgehäuse 29 angeordnet, welches hier ein Teilbereich des Ventilblocks 50 ist, und über den Ventilkörper 23 mit der Motorwelle 28 verbunden. Das Schaltventil 2 weist einen elektrisch angetriebenen Motor mit einem Stator und einen drehfest am Rotor befestigten Ventilkörper 23 auf. Der Motor weist zumindest eine im Betrieb stromdurchflossene Wicklung auf.

Der Ventilkörper 23 ist um eine Drehachse drehbar gelagert. Aufgrund der Drehachse ist eine Radial- und eine Axialrichtung des Ventilkörpers 23 definiert.

Von dem Ventilkörper 23 weisen zwei Schaltmittel radial nach außen. Die beiden Schaltmittel sind als symmetrische Ventil-Flächen 20 ausgebildet. Das Ventilgehäuse 29 weist einen Bewegungsraum für die Schaltmittel 20 auf und zumindest einen Anschlag 22 für die Schaltmittel. Der Anschlag ist so ausgeführt, dass er die Bewegung des Ventils so begrenzt, dass die Schaltmittel gegen den Anschlag stoßen. Die Rotation ist durch zwei Anschläge bevorzugt auf einen Bereich von 90 - 180 ° begrenzt. Das Umschalten zwischen Ein- und Ausatmung erfolgt durch zwei Schaltzustände. Eine Mittelstellung ist für den Schaltzustand Pause vorgesehen.

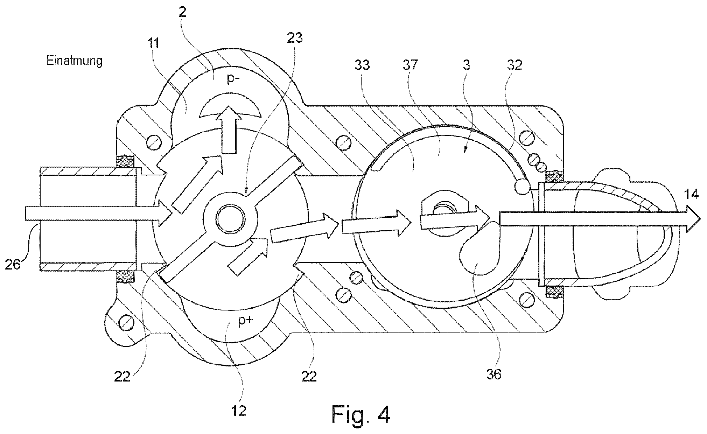

Figur 4 in Verbindung mit Fig. 2 und 3 zeigt: Das Schaltventil 2 und das Oszillationsventil 3 sind in dem Ventilblock 50 zwischen der Umgebung 15 und der Patientenleitung 14 in Reihe angeordnet. Die Gasleitung verbindet Schaltventil 2 und das Oszillationsventil 3 und ist in dem Ventilblock realisiert. Das Schaltventil 2 ist in dem Ventilgehäuse angeordnet, welches hier ein Teilbereich des Ventilblocks ist, und über den Ventilkörper 23 mit der Motorwelle verbunden. Das Schaltventil 2 weist einen elektrisch angetriebenen Motor mit einem Stator und einen drehfest am Rotor befestigten Ventilkörper 23 auf. Der Motor weist zumindest eine im Betrieb stromdurchflossene Wicklung auf. Der Ventilkörper 23 ist um eine Drehachse drehbar gelagert. Aufgrund der Drehachse 60 ist eine Radial- und eine Axialrichtung des Ventilkörpers 23 definiert.

Von dem Ventilkörper 23 weisen zwei Schaltmittel radial nach außen. Die beiden Schaltmittel sind als Ventil-Flächen ausgebildet. Das Ventilgehäuse weist einen Bewegungsraum für die Schaltmittel 20 und zumindest einen Anschlag 22 für die Schaltmittel auf. Der Anschlag ist so ausgeführt, dass er die Bewegung des Ventils so begrenzt, dass die Schaltmittel gegen den Anschlag stoßen. Die Rotation ist durch zwei Anschläge 22 bevorzugt auf einen Bereich von 90 - 180 ° begrenzt. Das Umschalten zwischen Ein- und Ausatmung erfolgt durch zwei Schaltzustände. Eine Mittelstellung ist für den Schaltzustand Pause vorgesehen.

Das Oszillationsventil ist bevorzugt als drehbar gelagertes Ventil ausgeführt und in dem Ventilgehäuse 39 angeordnet, welches hier ein Teilbereich des Ventilblocks 50 ist, und über den Ventilkörper 33 mit der Motorwelle verbunden. Das Oszillationsventil 3 weist einen Anschluss zur Patientenleitung 14 und einen Anschluss, zu dem Schaltventil 2 und einen Anschluss 36 zur Umgebungsluft auf. Das Schaltmittel ist in dieser Ausführungsform ein Drehventilkörper 33 mit Öffnungen in zwei unterschiedlichen Ebenen und Richtungen. Die Öffnungen weisen sowohl nach radial als auch nach axial. Zumindest ein Anschlag 32 am Ventil und/oder dem Gehäuse begrenzt die Rotation des Ventils. Eine Rotation um mehr als eine ganze Umdrehung wird so verhindert.

Das Oszillationsventil 3 weist einen elektrisch angetriebenen Motor mit einem Stator und einen drehfest am Rotor befestigten Ventilkörper 33 auf. Der Motor weist zumindest eine im Betrieb stromdurchflossene Wicklung auf. Der Ventilkörper 33 ist um eine Drehachse drehbar gelagert. Aufgrund der Drehachse ist eine Radial- und eine Axialrichtung des Ventilkörpers 33 definiert. Das Oszillationsventil und/oder der Ventilkörper 33 weist zumindest eine Öffnung auf, die zu der Patientenleitung führt eine Öffnung auf die zu der Umgebungsluft führt. Der Ventilkörper 33 ist so ausgestaltet, dass er, abhängig von der Ventilstellung, die Öffnung zur Patientenleitung oder die Öffnung zu der Umgebungsluft für einen Atemgasfluss zumindest teilweise verschließt oder öffnet.

Die Öffnung in Axialrichtung weist zum Anschluss 36 der zur Umgebungsluft 16 führt. Der Ventilkörper 33 weist einen zentralen Bereich auf, der sich zylinderförmig in Axialrichtung, um die Aufnahme für die Motorwelle, erstreckt. Der Ventilkörper 33 weist zudem eine Deckscheibe 37 auf, die sich ausgehend von dem oberen Ende des zentralen Bereiches in radialer Richtung 61 erstreckt.

Die Deckscheibe 37 ist nicht durchgehend ausgebildet und lässt zumindest eine Öffnung frei, die sich in Axialrichtung erstreckt.

Die Form der Öffnung 63 und oder die Form des Anschlusses 36 ist beispielsweise so gewählt, dass die Überlappung zwischen der Öffnung 63 und dem Anschluss 36, bei Rotation des Ventils, abhängig von der Drehrichtung linear anwächst oder abschwillt. Die Überlappung zwischen der Öffnung 63 und dem Anschluss 36, kann bei Rotation des Ventils auch nichtlinear anwachsen beispielsweise quadratisch oder logarithmisch oder unstetig.

Die Form der Öffnung 63 und oder die Form des Anschlusses 36 ist beispielsweise zumindest abschnittsweise oval, gerundet oder dreieckig.

Für die Einatmung gibt die Steuereinheit 4 die Stellung des Schaltventils 2 und des Oszillationsventils 3 so vor, dass ein Gasfluss von der Umgebung über die Anschlüsse 26 und 24 zur Saugseite 11 des Gebläses möglich ist und gleichzeitig ein Gasfluss von der Druckseite 12 über den Anschluss 25 und 27 zu dem Anschluss 35 des Oszillationsventils 3 und durch den Anschluss 34 zu der Patientenleitung 14. Das Schaltventil 2 und das Oszillationsventil 3 sind in dem Ventilblock zwischen dem Gebläse 10 und der Patientenleitung 14 in Reihe angeordnet.

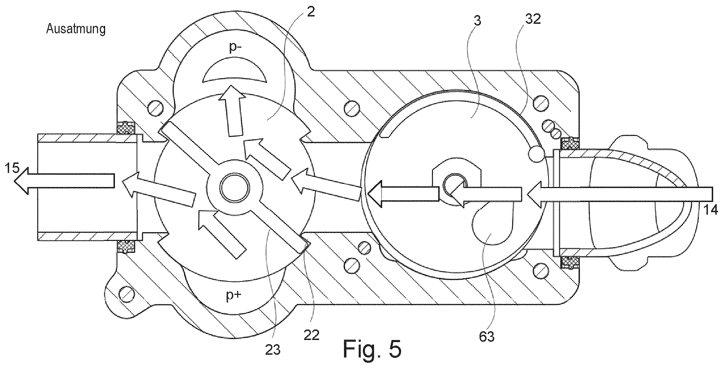

Figur 5 in Verbindung mit Fig. 2 und 3 zeigt: Für die Ausatmung gibt die Steuereinheit 4 die Stellung des Schaltventils 2 und des Oszillationsventil 3 so vor, dass ein Gasfluss von der Patientenleitung 14 über den Anschluss 34 und den Anschluss 35 des Oszillationsventils 3 über den Anschluss 27 und den Anschluss 24 des Schaltventils zur Saugseite 11 (P-) des Gebläses freigibt. Das Gebläse saugt somit Atemgas aus der Patientenleitung an. Das Atemgas durchströmt die Ventile 3 und 2 und den Anschluss 26 und tritt dann zur Umgebungsluft 15 aus. Der Ventilkörper 23 separiert mit seinen Schaltmitteln den Unterdruckbereich (P-) der angesaugten Gase (Pfeile), die von der Patientenleitung 14 durch das Ventil 3 strömen und durch einen Teil des Schaltventils 2 zur Saugseite des Gebläses gelangen, von dem Überdruckbereich (P+) der Gase, die von dem Gebläse beschleunigt werden und durch einen Teil des Schaltventils 2 zum Anschluss 26 und weiter zur Umgebungsluft 15 geführt werden. und einen Gasfluss von der Druckseite 12 zum Anschluss 26 führt und weiter zur Umgebungsluft 15 möglich ist. Im Vergleich zu der Stellung des Schaltventils 2 aus der Fig. 4 ist erkennbar, dass für die Ausatmung das Schaltventil so bewegt wurde, dass die von dem Ventilkörper 23 radial nach außen weisenden Schaltmittel an zwei andere Anschläge 22 anstoßen als in der Einatmungsstellung.

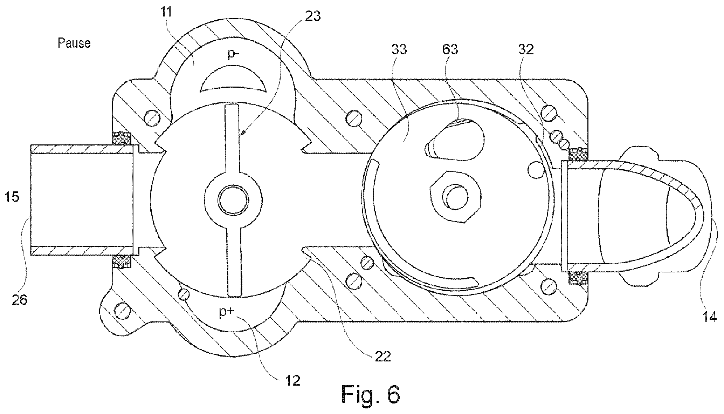

Figur 6 in Verbindung mit Fig. 2 und 3 zeigt: Im Vergleich zu der Stellung des Schaltventils 2 aus der Fig. 4 oder Fig. 5 ist erkennbar, dass für die Pause das Schaltventil so bewegt wurde, dass die von dem Ventilkörper 23 radial nach außen weisenden Schaltmittel an keinen der Anschläge 22 anstoßen. Vielmehr befinden sich die Schaltmittel in einer Stellung zwischen zwei Anschlägen 22. Durch diese Stellung der Schaltmittel ist ein Gasfluss von der Saugseite 11 des Gebläses 10 zu der Druckseite 12 frei.

Für die Pause gibt die Steuereinheit 4 die Stellung des Schaltventils 2 und des Oszillationsventils 3 so vor, dass ein Gasfluss durch den Anschluss 25 zu dem Anschluss 24 frei ist und so ein Gasfluss von der Saugseite 11 des Gebläses 10 zu der Druckseite 12 frei ist und ein Gasfluss zu der Umgebung zumindest teilweise unterbunden ist oder nicht aktiv durch das Gebläse befördert wird. Dadurch, dass in dieser Schaltstellung eine Rezirkulation von der Druckseite des Gebläses hin zur Saugseite des Gebläses stattfindet, wird auch kein Gasfluss hin zum Oszillationsventil oder hin zum Patienten oder weg vom Oszillationsventil oder weg vom Patienten erzwungen. Das Schaltventil ist vielmehr bevorzugt derart gestaltet, dass es in der Schaltstellung Pause bezüglich der Patientenatmung neutral wirkt und eine Ein- oder Ausatmung seitens des Patienten durch das Schaltventil grundsätzlich möglich ist.

Das Oszillationsventils 3 wurde so geschaltet, dass ein Gasfluss von oder zum Schaltventil nicht möglich ist. Bevorzugt ein eine freie Atmung des Patienten durch das Oszillationsventil zu der Umgebung möglich, da das Ventil so bewegt wurde, dass die Öffnung 63 mit dem Anschluss 36 zur Umgebungsluft gasleitend verbunden ist und so eine Gasfluss zwischen dem Patienten und der Umgebung möglich ist.

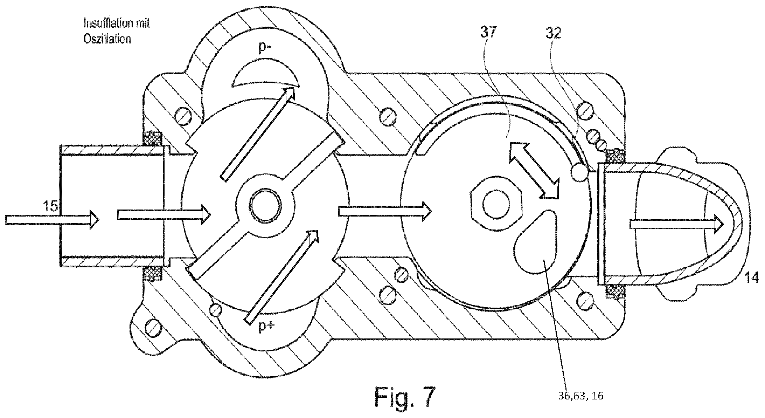

Figur 7 in Verbindung mit Fig. 2-5 zeigt: Für die Einatmung mit Oszillation gibt die Steuereinheit 4 die Stellung des Schaltventils 2 und des Oszillationsventils 3 so vor, dass ein Gasfluss (Pfeile) von der Umgebung 15 über die Anschlüsse 26 und 24 zur Saugseite 11 des Gebläses möglich ist und gleichzeitig ein Gasfluss von der Druckseite 12 über den Anschluss 25 und 27 zu dem Anschluss 35 des Oszillationsventils 3 und durch den Anschluss 34 zu der Patientenleitung 14. Zudem gibt die Steuereinheit 4 die Stellung des Oszillationsventils 3 so vor, dass alternierend der Anschluss 36 mit der Umgebung verbunden und wieder nicht verbunden ist (angedeutet durch den Doppelpfeil) . Abhängig von der Frequenz mit der die Stellung des Oszillationsventils 3 in dieser Weise vorgibt, ergeben sich Fluss- und Druckschwankungen (Oszillationen), die den Atemluftstrom zum Patienten überlagern. Durch die alternierende Verbindung mit der Umgebungsluft ergibt sich ein pulsierender Druckabbau, der mit der Frequenz der Bewegung des Oszillationsventils 3 korrespondiert.

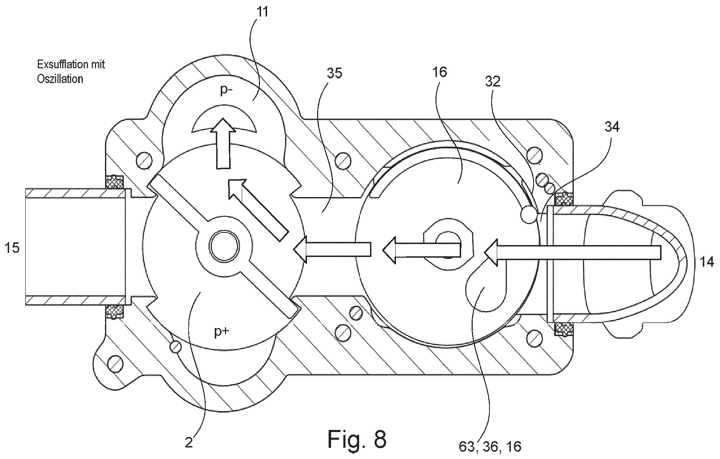

Figur 8 zeigt in Verbindung mit Fig. 2-5 und 7: Für die Ausatmung mit Oszillation gibt die Steuereinheit 4 die Stellung des Schaltventils 2 und des Oszillationsventils 3 so vor, dass ein Gasfluss von der Patientenleitung 14 über den Anschluss 34 und den Anschluss 35 des Drehventils 3 über den Anschluss 27 und den Anschluss 24 des Schaltventils zur Saugseite 11 des Gebläses (P-) freigibt und einen Gasfluss von der Druckseite 12 (P+) zu der Umgebung 15 möglich ist. Zudem gibt die Steuereinheit 4 die Stellung Oszillationsventils 3 so vor, dass alternierend der Anschluss 36 mit der Umgebung verbunden und wieder nicht verbunden ist. Abhängig von der Frequenz mit der die die Stellung Oszillationsventils 3 in dieser Weise vorgibt, ergeben sich Fluss- und Druckschwankungen, die den Atemluftstrom der Ausatmung des Patienten überlagern. Durch die alternierende Verbindung mit der Umgebungsluft ergibt sich ein pulsierender Druckabbau, der mit der Frequenz der Bewegung des Oszillationsventils 3 korrespondiert.

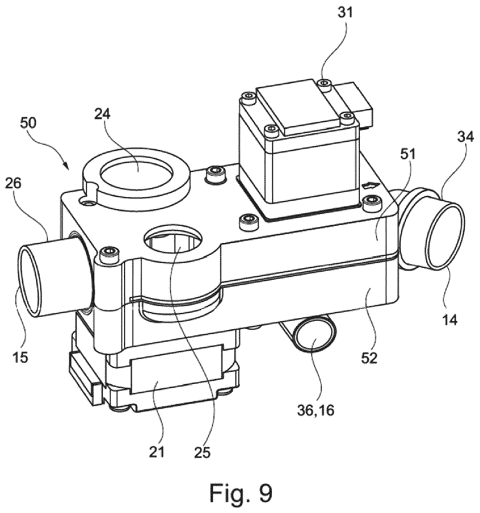

Figur 9 zeigt in Verbindung mit Fig. 2-8: Das offenbarungsgemäße Ventil 2, 3 ist hier als ein gemeinsamer Ventilblock 50 aufgebaut. Die hier nicht dargestellte Gasleitung 13, welche die Druckseite 12 des Gebläses gasleitend mit einer Patientenleitung 14 verbindet ist in dem Ventilblock realisiert. Die Gasleitung 13 ist mit einem Schaltventil 2 und einem Oszillationsventil 3 verbunden. Das Schaltventil 2 weist einen Anschluss 24 zur Saugseite 11 und einen Anschluss 25 zur Druckseite 12 des nicht gezeigten Gebläses auf, einen Anschluss 26 zur Umgebungsluft 15 und einen Anschluss 27 zur Patientenleitung 14 auf. Das Oszillationsventil 3 weist einen Anschluss 34 zur Patientenleitung 14 und einen Anschluss 35, zu dem Schaltventil 2 und einen Anschluss 36 zur Umgebungsluft 16 auf.

Das Schaltventil 2 und das Oszillationsventil 3 sind in dem Ventilblock zwischen dem Gebläse 10 und der Patientenleitung 14 in Reihe angeordnet. Der Anschluss 27 des Schaltventils 2 ist über die Gasleitung 13 mit dem Anschluss 35 des Oszillationsventils 3 verbunden und der Anschluss 34 ist mit der Patientenleitung 14 verbunden. Das Gebläse ist insofern über das Schaltventil 2 und das Oszillationsventil 3 mittelbar mit der Patientenleitung 14 verbunden. Der Ventilblock 50 weist eine Oberhälfte 51 und eine Unterhälfte 52 auf. Im Bereich der Oberhälfte 51 ist der Motor 31 des Oszillationsventils angeordnet. Im Bereich der Unterhälfte 52 ist der Motor 21 des Schaltventils angeordnet. Die Motoren oder können auch auf einer Seite oder einer Hälfte 51, 52 angeordnet sein.

Oberhälfte 51 und Unterhälfte 52 können miteinander verschaubt oder verrastet oder verklebt sein. Die Realisierung der Ventilkörper beider Ventile in einem Ventilgehäuse hat wiederum Vorteile hinsichtlich Herstellkosten und Bauraum.

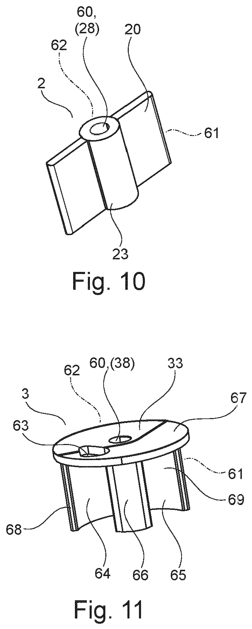

Figur 10 zeigt: Der Ventilkörper 23 ist um eine Drehachse 60 drehbar gelagert. Aufgrund der Drehachse 60 ist eine Radial- und eine Axialrichtung 61, 62 des Ventilkörpers 23 definiert.

Von dem Ventilkörper 23 weisen zwei Schaltmittel 20 radial nach außen. Die beiden Schaltmittel 20 sind als symmetrische Ventil-Flächen 20 ausgebildet. Das Ventilgehäuse 29 weist einen Bewegungsraum für die Schaltmittel 20 auf und zumindest einen Anschlag 22 für die Schaltmittel. Der Anschlag ist so ausgeführt, dass er die Bewegung des Ventils so begrenzt, dass die Schaltmittel gegen den Anschlag stoßen. Die Rotation ist durch zwei Anschläge bevorzugt auf einen Bereich von 90 - 180 ° begrenzt. Das Umschalten zwischen Ein- und Ausatmung erfolgt durch zwei Schaltzustände. Eine Mittelstellung ist für den Schaltzustand Pause vorgesehen.

Fig. 11 zeigt: Das Oszillationsventil ist bevorzugt als drehbar gelagertes Ventil ausgeführt und in dem Ventilgehäuse 39 angeordnet, welches hier ein Teilbereich des Ventilblocks 50 ist, und über den Ventilkörper 33 mit der Motorwelle 38 verbunden. Das Oszillationsventil 3 weist einen Anschluss 34 zur Patientenleitung 14 und einen Anschluss 35, zu dem Schaltventil 2 und einen Anschluss 36 zur Umgebungsluft 16 auf. Das Schaltmittel ist in dieser Ausführungsform ein Drehventilkörper 33 mit Öffnungen 63,64,65 in zwei unterschiedlichen Ebenen und Richtungen. Die Öffnungen weisen sowohl nach radial als auch nach axial. Zumindest ein Anschlag 32 am Ventil und/oder dem Gehäuse begrenzt die Rotation des Ventils. Eine Rotation um mehr als eine ganze Umdrehung wird so verhindert.

Das Oszillationsventil weist einen elektrisch angetriebenen Motor 21 mit einem Stator und einen drehfest am Rotor befestigten Ventilkörper 33 auf. Der Motor weist zumindest eine im Betrieb stromdurchflossene Wicklung auf.

Der Ventilkörper 33 ist um eine Drehachse 60 drehbar gelagert. Aufgrund der Drehachse 60 ist eine Radial- und eine Axialrichtung 61, 62 des Ventilkörpers 33 definiert.

Das Oszillationsventil und/oder der Ventilkörper 33 weist zumindest eine Öffnung auf, die zu der Patientenleitung führt eine Öffnung 63 auf die zu der Umgebungsluft führt und der der Ventilkörper 33 ist so ausgestaltet, dass der, abhängig von der Ventilstellung, die Öffnung, die zu der Patientenleitung führt oder die Öffnung 63, die zu der Umgebungsluft führt für einen Atemgasfluss zumindest teilweise verschließt oder öffnet.

Das Oszillationsventil und/oder der Ventilkörper 33 weist zumindest eine Öffnung auf, die zu der Patientenleitung führt und eine weitere Öffnung die zu der Umgebungsluft führt und wobei der der Ventilkörper 33 so ausgestaltet ist, dass er, abhängig von der Ventilstellung, die Öffnung, die zu der Patientenleitung führt oder die Öffnung, die zu der Umgebungsluft führt, für einen Atemgasfluss zumindest teilweise verschließt oder öffnet.

Der Ventilkörper 33 des Oszillationsventils weist einen Bereich 66 auf, der die Aufnahme 38 für die Motorwelle darstellt und zudem einen Wandbereich 68 auf, der in einer Drehstellung des Ventils die Öffnung, die zu der Patientenleitung führt oder die Öffnung, die zu der Umgebungsluft führt, für einen Atemgasfluss zumindest teilweise verschließt oder öffnet und entsprechend der Drehstellung des Ventils somit zumindest eine Öffnung 63,64,65 des Ventils einen Atemgasfluss in Richtung der Patientenleitung freigibt oder einen Gasfluss in Richtung der Umgebung 16.

Die Öffnung 63 in Axialrichtung 62 weist zum Anschluss 36 der zur Umgebungsluft 16 führt. Das Oszillationsventil und/oder der Ventilkörper 33 weist zumindest eine Öffnung 65,64 in Radialrichtung 61 und zumindest eine Öffnung 63 in Axialrichtung 62 auf. Die Öffnung 63 in Axialrichtung 62 weist zum Anschluss 36 der zur Umgebungsluft 16 führt. Der Ventilkörper 33 weist einen zentralen Bereich 66 auf, der sich zylinderförmig in Axialrichtung 62, um die Aufnahme 38 für die Motorwelle, erstreckt. Der Ventilkörper 33 weist zudem eine Deckscheibe 37 auf, die sich ausgehend von dem oberen Ende des zentralen Bereiches 66 in radialer Richtung 61 erstreckt. An der radialen Außenkante oder nahe der Außenkante der Deckscheibe 37 weist der Ventilkörper einen Wandbereich 68 auf, der sich in axialer Richtung 62 oder in einem rechten Winkel von der Deckscheibe ausgehend und im Wesentlichen parallel zu dem zentralen Bereich 66 erstreckt. Zwischen dem Wandbereich 68 und dem zentralen Bereich 66 erstreckt sich ein Kanal 69 der gasführend ist und sich zumindest zwischen den Öffnungen 65,64 erstreckt und/oder auch mit der Öffnung 63 kommuniziert. Der Kanal 69 weist so beispielsweise drei Öffnungen 63, 64,65 auf, die in axiale 62 und/oder radiale 61 Richtung weisen.

Die Deckscheibe 37 ist nicht durchgehend ausgebildet und lässt zumindest eine Öffnung 63 frei, die sich in Axialrichtung 62 erstreckt.

Der Wandbereich 68 ist nicht durchgehend ausgebildet und lässt zumindest zwei Öffnung 65,64 frei, die sich in Radialrichtung 61 erstrecken. Die zumindest eine Öffnung 65,64 in Radialrichtung 61 kann auch als eine Bohrung durch den Ventilkörper ausgeführt sein. Es liegen dann zwei Öffnung 65,64 vor die durch einen Kanal 69 miteinander verbunden sind. Eine Öffnung kann im Sinne der Erfindung auch ein weitlumiger Bereich sein, der einen hohen Gasfluss ermöglicht. Wobei der Wandbereich jede Form annehmen kann, die geeignet ist einen Gasfluss im Wesentlichen zu verhindern. Der Wandbereich kann daher eine Fläche einnehmen, die kleiner ist als die Fläche der Öffnung.

Die Form der Öffnung 63 und oder die Form des Anschlusses 36 ist beispielsweise so gewählt, dass die Überlappung zwischen der Öffnung 63 und dem Anschluss 36, bei Rotation des Ventils, abhängig von der Drehrichtung linear anwächst oder abschwillt. Die Überlappung zwischen der Öffnung 63 und dem Anschluss 36, kann bei Rotation des Ventils auch nichtlinear anwachsen beispielsweise quadratisch oder logarithmisch oder unstetig.

Die Form der Öffnung 63 und oder die Form des Anschlusses 36 ist beispielsweise zumindest abschnittsweise oval, gerundet oder dreieckig.

Das Oszillationsventil ermöglicht eine Oszillation auf dem Drucksignal durch Abbauen des Drucks über eine Öffnung 63 zur Umgebung. Die Öffnung zur Umgebung kann zusätzlich zur Entlastung des Patienten genutzt werden. Die Öffnung zur Umgebung kann darüber hinaus das Atmen des Patienten in der Schaltstellung Pause unterstützen oder erleichtern.

Es können auch andere als die hier gezeigten Ventilstellungen für die Oszillation während der Insufflation und/oder Exsufflation vorgesehen sein.

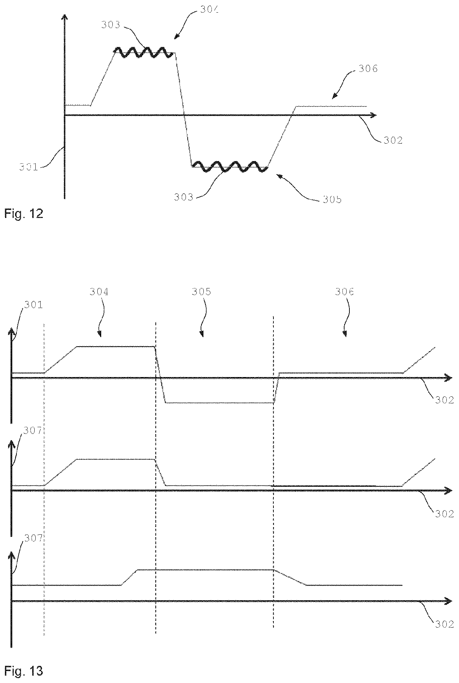

Die Figur 12 zeigt ein Beispiel eines Druckverlaufs, wie er bei einem Einsatz des Beatmungsgeräts 1 vorgesehen sein kann. Dazu wurde der Druck 301 gegen die Zeit 302 aufgetragen.

Während der Insufflation 304 liegt für eine definierte Zeit ein entsprechend hoher Druck 301 vor. Für eine besonders wirksame Anregung des Hustenreizes bzw. zum besonders wirksamen Lösen des Sekrets wird dann sehr kurzfristig auf die Exsufflation 305 umgeschaltet. Dazu wird der Druck 301 innerhalb einer definierten Zeitspanne auf ein entsprechend negatives Niveau abgesenkt und für eine bestimmte Dauer gehalten. Die Umschaltung erfolgt offenbarungsgemäß insbesondere auch durch das Schaltventil 2 und das Gebläse.

Dann kann beispielsweise eine erneute Anhebung des Drucks auf das gewünschte Niveau für die Insufflation erfolgen. Die Umschaltung erfolgt offenbarungsgemäß auch hier insbesondere auch durch das Schaltventil 2 und das Gebläse. Anschließend erfolgt für die Exsufflation wieder eine sehr zügige Absenkung des Drucks 301. Dieses Wechseln zwischen Insufflation und Exsufflation kann für einen gewünschten Zeitraum wiederholt werden. Beispielsweise kann die Anzahl der Wiederholungen und/oder die Frequenz der Wiederholungen durch einen Benutzer bzw. Betreuer vorgegeben werden.

In dem hier gezeigten Verlauf ist nach der Exsufflation 305 eine Pause 306 vorgesehen. Das bietet dem Patienten eine große Erleichterung, da die Hustenvorgänge eine erhebliche körperliche Anstrengung verlangen. Der hier gezeigte Druckverlauf weist während der Pause 306 einen leichten Überdruck bzw. einen positiven Therapiedruck auf. Das Ausatmen gegen einen solchen leichten, gezielten Überdruck ist atemtherapeutisch besonders sinnvoll. Der Überdruck kann beispielsweise als ein konstanter positiver Druck (CPAP) ausgebildet sein. Beispielsweise liegt der Druck zwischen 4 und 30 mbar. Hingegen kann für die Exsufflation und/oder Insufflation ein Druck im Bereich von etwa +/-70 mbar oder auch höher eingestellt werden. In der Pause ergeben sich im Rahmen von Ein- und Ausatmung typischerweise erheblich kleinere Flows im Vergleich zur In- oder Exsufflation.

In der Pause kann auch eine Beatmung vorgesehen sein. Beispielsweise ist dann für die Beatmung bzw. Inspiration ein Druck bis etwa 50 mbar und insbesondere zwischen 10-35 mbar vorgesehen.

Der Abfall des Drucks 301 bei dem Übergang von Insufflation zu Exsufflation erfolgt hier vorzugsweise durch ein entsprechend zügiges Umschalten der Ventileinheit. Die Drehzahl des Gebläses für die Exsufflation wird vorzugsweise bereits vor dem Schaltvorgang der Ventileinheit entsprechend angepasst. Offenbarungsgemäß ist dies aber nicht notwendig.

Der Anstieg des Drucks 301 von der Exsufflation zur nächsten Insufflation oder nach einer Pause hin zur folgenden Insufflation erfolgt vorzugsweise weniger zügig bzw. über einen längeren Zeitraum. Der Druckanstieg kann neben der Änderung der Ventilstellung durch ein entsprechend behutsames Hochfahren des Gebläses erfolgen.

Auch die Erhöhung des Drucks 301 im Vorlauf für die Pause 306 erfolgt hier durch eine entsprechend langsame Drehzahlerhöhung des Gebläses.

Auf dem Niveau der Inspiration 304 oder dem der Exspiration 305 oder in der Umschaltphase zwischen Inspiration 304 und Exspiration 305 oder während der Pause 306 kann erfindungsgemäß eine definierte Oszillation erfolgen. Das Oszillationsventil 3 wirkt dabei in der Weise, dass durch schrittweises Öffnen und Schließen dieses Ventils der Strömungswiderstand in der Gasleitung zwischen Gasquelle und Patient in der Art variiert werden kann, dass Oszillationen von Fluss und/oder Druck bewirkt werden.

Die Figur 13 zeigt ein Beispiel für ein Hustenmanöver mit einer anschließenden Pause 306. Dazu wurde in der oberen Grafik der Druck 301 gegen die Zeit 302 aufgetragen. In der mittleren Grafik wurde eine Drehzahl 307 des Gebläses beispielhaft und stark idealisiert gegen die Zeit 302 aufgetragen. In der unteren Grafik wurde eine Drehzahl 307 des Gebläses beispielhaft und stark idealisiert gegen die Zeit 302 aufgetragen. Die vertikal verlaufenden, gestrichelten Linien deuten hierbei stark schematisiert ein Umschalten der Ventilstellung an. Zu Beginn des Manövers wird die Ventileinheit in die Ventilstellung für die Insufflation gebracht.

Dann wird die Drehzahl 307 des Gebläses über eine definierte Zeit langsam erhöht. Entsprechend erhöht sich der Druck 301. Nach Erreichen des für die Insufflation erforderlichen Drucks 301 wird die Drehzahl 307 gehalten.

Nach einer bestimmten Zeit erfolgt der Wechsel von der Insufflation 304 zu Exsufflation 305. Für ein wirksames Auslösen des Hustenreizes bzw. für eine besonders wirksame Unterstützung der Sekretabfuhr erfolgt der Wechsel hier besonders kurzfristig. Dazu wird die Ventileinheit in die zweite Ventilstellung geschaltet. Der Druck 301 fällt über einen sehr kurzen Zeitraum entsprechend ab. Der für die Exsufflation 305 notwendige negative Druck wird erreicht.

Um den Druckübergang besonders kurzfristig ermöglichen zu können, wurde die Drehzahl 307 bereits vor dem Zuschalten auf das erforderliche Maß erhöht. Der Druck 301 bzw. die Drehzahl für die Exsufflation 305 werden nun für eine vorbestimmte Zeit gehalten.

Anschließend erfolgt wieder ein Umschalten der Ventileinheit. Nach dem Zuschalten wird die Drehzahl 307 soweit erhöht, dass ein entsprechend leichter und für die Beatmung während der Pause 306 geeigneter Überdruck vorliegt. Das Gebläse beschleunigt also während des Druckaufbaus bzw. zur Erzeugung des Druckverlaufs.

Nach dem Ende der Pause 306 kann wieder eine Erhöhung der Drehzahl erfolgen, um den für die Insufflation 304 benötigten Druck 301 zu erreichen. Das Hustenmanöver kann nun von Neuem beginnen.

Insgesamt bietet die hier vorgestellte Offenbarung den Vorteil, dass eine besonders patientenfreundliche und zugleich wirksame Hustenmaschine bereitgestellt wird. Zudem bietet die Offenbarung den Vorteil, dass auch eine erheblich verbesserte Beatmung möglich ist.

So kann während der Beatmung z. B. eine besonders sanfte Unterstützung bei der Sekretabfuhr erfolgen, in dem der Patient in einer Ausatemphase mit einem negativen Therapiedruck unterstützt wird. Besonders vorteilhaft kann die Offenbarung dabei mit einem Zweischlauchsystem eingesetzt werden.

Ein weiterer Vorteil ist, dass die Beatmung alleine oder auch in Kombination mit einer Husten- bzw. Sekrettherapie erfolgen kann. Beispielsweise erfolgt während einer Husten- bzw. Sekrettherapie eine Pause, in welcher ein positiver Therapiedruck eingesetzt wird, um den Patienten zu entlasten. In der Pause kann auch eine Beatmung des Patienten erfolgen.

## Patentansprüche

1. Beatmungsgerät (1) mit einer Gasquelle (10), zumindest einer Gasleitung (9, 11, 12, 13) und einer Patientenleitung (14) und zumindest zwei Ventilen (2, 3, 50) wobei jedes der Ventile zumindest mittelbar einen Anschluss zur Umgebungsluft (15, 16) aufweist und wobei die Ventile in der Gasleitung oder als Teil der Gasleitung angeordnet sind, wobei die Ventile (2,3) zumindest zeitweise gasleitend mit der Gasquelle (10) und der Patientenleitung (14) oder der Umgebungsluft (15, 16) verbunden sind, wobei eines der Ventile ein Schaltventil (2) ist, wobei das andere Ventil (3) ein Oszillationsventil ist und in der Weise wirkt, dass durch schrittweises Öffnen und Schließen des Ventils der Strömungswiderstand in der Gasleitung zwischen Gebläse und Patient variiert werden kann, wodurch während der Insufflation und/oder der Exsufflation Oszillationen von Fluss und Druck bewirkt werden, wobei das Oszillationsventil (3) zumindest eine Öffnung aufweist, die zu der Patientenleitung führt und eine weitere Öffnung die zu der Umgebungsluft führt und wobei das Ventil (3) so ausgestaltet ist, dass es, abhängig von der Ventilstellung, die Öffnung, die zu der Patientenleitung führt oder die Öffnung, die zu der Umgebungsluft führt, für einen Atemgasfluss zumindest teilweise verschließt oder öffnet, wobei das Oszillationsventil (3) als Drehventil mit einem Drehventilkörper (33), einem Motor (31) und mit Öffnungen (63,64,65) in mehreren Ebenen in einem Ventilgehäuse (39) ausgebildet ist, wobei in einem Schaltzustand Pause eine Rotation des Drehventilkörpers (33) die Öffnung (63) mit einer Öffnung zur Umgebung (36) freigibt und einen Gasfluss oder einen Druckabbau in die Umgebung (16) ermöglicht und die Patientenleitung (14) direkt, unter Umgehung des Schaltventils (2), mit der Umgebung verbunden ist.

2. Beatmungsgerät (1) nach zumindest einem der vorhergehenden Ansprüche **dadurch gekennzeichnet, dass** zumindest eines der Ventile (2,3) als drehbar gelagertes Ventil ausgeführt ist und/oder dass zumindest eines der Ventile (2,3) als Axialventil ausgeführt ist.

3. Beatmungsgerät (1) nach zumindest einem der vorhergehenden Ansprüche **dadurch gekennzeichnet, dass** das Oszillationsventil (3) einen elektrisch angetriebenen Motor (21) mit einem Stator und einen drehfest am Rotor befestigten Ventilkörper (33) aufweist wobei der Ventilkörper (33) um eine Drehachse (60) drehbar gelagert ist und aufgrund der Drehachse (60) eine Radial- und eine Axialrichtung (61, 62) des Ventilkörpers (33) definiert sind.

4. Beatmungsgerät (1) nach zumindest einem der vorhergehenden Ansprüche **dadurch gekennzeichnet, dass** der Ventilkörper (33) des Oszillationsventils (3) einen Bereich (66) aufweist, der die Aufnahme (38) für die Motorwelle darstellt und zudem einen Wandbereich (68) aufweist, der in einer Drehstellung des Ventils die Öffnung, die zu der Patientenleitung führt oder die Öffnung, die zu der Umgebungsluft führt, für einen Atemgasfluss zumindest teilweise verschließt oder öffnet und entsprechend der Drehstellung des Ventils somit zumindest eine Öffnung (63,64,65) des Ventils einen Atemgasfluss in Richtung der Patientenleitung freigibt oder einen Gasfluss in Richtung der Umgebung (16).

5. Beatmungsgerät (1) nach zumindest einem der vorhergehenden Ansprüche **dadurch gekennzeichnet, dass** das Oszillationsventil (3) als drehbar gelagertes Ventil ausgeführt ist und der Ventilkörper (33) zumindest eine Öffnung (65,64) in Radialrichtung (61) und zumindest eine Öffnung (63) in Axialrichtung (62) aufweist, wobei die Öffnung (63) in Axialrichtung (62) zum Anschluss (36) der zur Umgebungsluft (16) führt weist.

6. Beatmungsgerät (1) nach zumindest einem der vorhergehenden Ansprüche **dadurch gekennzeichnet, dass** der Ventilkörper (33) des Oszillationsventils einen zentralen Bereich (66) aufweist, der sich zylinderförmig in Axialrichtung (62) um die Aufnahme (38) für die Motorwelle, erstreckt und der Ventilkörper (33) zudem eine Deckscheibe (37) aufweist, die sich ausgehend von dem oberen Ende des zentralen Bereiches (66) in radialer Richtung (61) erstreckt und an der radialen Außenkante oder nahe der Außenkante der Deckscheibe (37) ein Wandbereich (68), der sich in axialer Richtung (62) oder in einem rechten Winkel von der Deckscheibe ausgehend und im Wesentlichen parallel zu dem zentralen Bereich (66) erstreckt, wobei sich zwischen dem Wandbereich (68) und dem zentralen Bereich (66) ein Kanal (69) der gasführend ist erstreckt und die Öffnungen (63,64,65) über den Kanal gasführend verbunden sind.

7. Beatmungsgerät (1) nach zumindest einem der vorhergehenden Ansprüche **dadurch gekennzeichnet, dass** die Form der Öffnung (63) und oder die Form des Anschlusses (36) so ausgeführt sind, dass die Überlappung zwischen der Öffnung (63) und dem Anschluss (36), bei Rotation des Ventils je nach Drehrichtung, linear anwächst oder abschwillt.

8. Beatmungsgerät nach zumindest einem der vorhergehenden Ansprüche **dadurch gekennzeichnet, dass** die Steuereinheit (4) eingerichtet und ausgebildet ist, das Gebläse (10) zu steuern und/oder das Schaltventil (2) zu steuern und/oder das Oszillationsventil (3) zu steuern, wobei für die Insufflation durch das Gebläse für eine definierte Zeit ein entsprechend hoher Druck (301) vorgegeben wird und dann durch das Schaltventil (2) und/oder das Gebläse (10) auf die Exsufflation (305) umgeschaltet wird wozu der Druck (301) innerhalb einer definierten Zeitspanne auf ein entsprechend negatives Niveau abgesenkt und für eine bestimmte Dauer gehalten wird, wobei anschließend durch das Gebläse eine erneute Anhebung des Drucks auf das gewünschte Niveau für die Pause (306) erfolgt, wobei der Druck während der Pause (306) einen leichten Überdruck aufweist, welcher insbesondere zwischen 2 und 15 mbar liegt.

9. Beatmungsgerät (1) nach zumindest einem der vorhergehenden Ansprüche **dadurch gekennzeichnet, dass** das der Motor (21) den Ventilkörper (23) zwischen zumindest drei Schaltzuständen rotiert, wobei die maximale Rotation zwischen den Anschlägen maximal 150 ° ist, bevorzugt maximal 120°, besonders bevorzugt maximal 100° ist und/oder wobei die Schaltzustände für das Schaltventil (2) Einatmung, Ausatmung und Pause sind, wobei für die Einatmung die Stellung des Ventilkörpers (23) einen Gasfluss von der Umgebung zur Saugseite des Gebläses freigibt und gleichzeitig einen Gasfluss von der Druckseite (12) über den Anschluss (27) zu dem Anschluss (35) des zweiten Ventils (3) ermöglicht.

10. Beatmungsgerät nach zumindest einem der vorhergehenden Ansprüche **dadurch gekennzeichnet, dass** die Schaltzustände für das Schaltventil (2) Einatmung, Ausatmung und Pause sind, wobei für die Pause die Stellung des Ventilkörpers (23) einen Gasfluss von der Saugseite (11) des Gebläses (10) zu der Druckseite (12) freigibt wodurch der Gasfluss in mindestens einer der beiden Richtungen von der Umgebung (15) hin zum Patienten (14) oder vom Patienten hin zur Umgebung im Wesentlichen unterbunden wird.

11. Beatmungsgerät (1) nach zumindest einem der vorhergehenden Ansprüche **dadurch gekennzeichnet, dass** zumindest ein Anschlag (32) an dem Ventilgehäuse (39) ausgebildet ist.

12. Beatmungsgerät (1) nach zumindest einem der vorhergehenden Ansprüche **dadurch gekennzeichnet, dass** der Drehventilkörper (33) Öffnungen (63,64,65), aufweist, die in radiale Richtung (61) und axiale Richtung (62) weisen, wobei
die Öffnung (63) zur Umgebungsluft axial in der Deckscheibe (37) des Drehventilkörpers angeordnet ist und eine Rotation des Drehventilkörpers die Öffnung (63) in Überdeckung mit dem Anschluss (36) zur Umgebungsluft bringt.

13. Beatmungsgerät (1) nach zumindest einem der vorhergehenden Ansprüche **dadurch gekennzeichnet, dass** die Öffnungen (64,65) radial im Drehventilkörper angeordnet sind und, in der entsprechenden Schaltstellung des Drehventilkörpers, einen Gasfluss von der Druckseite (12) über den Anschluss (27) des Schaltventils (2) zu dem Anschluss (35) des zweiten Ventils (3) und weiter über den Anschluss (34) zu der Patientenleitung (14) ermöglichen.

## Claims

1. A ventilator (1) with a gas source (10), at least one gas line (9, 11, 12, 13) and one patient line (14), and at least two valves (2, 3, 50), wherein each of the valves has, at least indirectly, a connection to the ambient air (15, 16) and wherein the valves are arranged in the gas line or as part of the gas line, wherein the valves (2, 3) are connected at least temporarily in a gas-conducting manner to the gas source (10) and the patient line (14) or to the ambient air (15, 16), wherein one of the valves is a switching valve (2), wherein the other valve (3) is an oscillating valve and acts such that the flow resistance in the gas line between the blower and the patient can be varied by opening and closing the valve in steps, as a result of which oscillations of flow and pressure are caused during the insufflation and/or the exsufflation, wherein the oscillating valve (3) has at least one opening that leads to the patient line and another opening that leads to the ambient air and wherein the valve (3) is designed such that, depending on the position of the valve, it at least partially closes or opens the opening that leads to the patient line or the opening that leads to the ambient air for a flow of respiratory gas, wherein the oscillating valve (3) is designed as a rotary valve with a rotary valve body (33), a motor (31), and with openings (63, 64, 65) in multiple levels in a valve housing (39), wherein, in a paused switching state, a rotation of the rotary valve body (33) releases the opening (63) with an opening to the environment (36) and enables a flow of gas or a reduction in pressure into the environment (16) and the patient line (14) is connected directly, by bypassing the switching valve (2), to the environment.

2. The ventilator (1) according to at least one of the preceding claims, **characterized in that** at least one of the valves (2, 3) is implemented as a rotatably mounted valve and/or **in that** at least one of the valves (2, 3) is implemented as an axial valve.

3. The ventilator (1) according to at least one of the preceding claims, **characterized in that** the oscillating valve (3) has an electrically driven motor (21) with a stator and a valve body (33) fastened to the rotor so as to be rotationally fixed, wherein the valve body (33) is mounted so as to be rotatable about an axis of rotation (60) and, due to the axis of rotation (60), a radial direction and an axial direction (61, 62) of the valve body (33) are defined.

4. The ventilator (1) according to at least one of the preceding claims, **characterized in that** the valve body (33) of the oscillating valve (3) has a region (66) which forms a receiving area (38) for the motor shaft and additionally has a wall region (68) which, in a rotational position of the valve, at least partly closes or opens the opening that leads to the patient line or the opening that leads to the ambient air for a flow of respiratory gas and, corresponding to the rotational position of the valve, thus releases at least one opening (63, 64, 65) of the valve for a flow of respiratory gas in the direction of the patient line or a flow of gas in the direction of the environment (16).

5. The ventilator (1) according to at least one of the preceding claims, **characterized in that** the oscillating valve (3) is implemented as a rotatably mounted valve and the valve body (33) has at least one opening (65, 64) in the radial direction (61) and at least one opening (63) in the axial direction (62), wherein the opening (63) in the axial direction (62) faces the connection (36) that leads to the ambient air (16).

6. The ventilator (1) according to at least one of the preceding claims, **characterized in that** the valve body (33) of the oscillating valve has a central region (66) which extends in the shape of a cylinder in the axial direction (62) about the receiving area (38) for the motor shaft, and the valve body (33) additionally has a cover disk (37) which extends in the radial direction (61) starting from the upper end of the central region (66) and, on the radial outer edge or near the outer edge of the cover disk (37), a wall region (68) which extends in the axial direction (62) or at a right angle starting from the cover disk and substantially parallel to the central region (66), wherein a gas-conducting channel (69) extends between the wall region (68) and the central region (66) and the openings (63, 64, 65) are connected in a gas-conducting manner via the channel.

7. The ventilator (1) according to at least one of the preceding claims, **characterized in that** the shape of the opening (63) and/or the shape of the connection (36) are implemented such that the overlap between the opening (63) and the connection (36) grows or shrinks linearly as the valve rotates, depending on the direction of rotation.

8. The ventilator according to at least one of the preceding claims, **characterized in that** the control unit (4) is configured and designed to control the blower (10) and/or to control the switching valve (2) and/or to control the oscillating valve (3), wherein a correspondingly high pressure (301) is specified for a defined time for the insufflation by the blower and then the switching valve (2) and/or the blower (10) switches to the exsufflation (305), for the purpose of which the pressure (301) is lowered to a correspondingly negative level within a defined period of time and is held for a specific duration, wherein an increase again of the pressure to the desired level for the pause (306) then takes place by means of the blower, wherein the pressure during the pause (306) has a slight overpressure, which is in particular between 2 and 15 mbar.

9. The ventilator (1) according to at least one of the preceding claims, **characterized in that** the motor (21) rotates the valve body (23) between at least three switching states, wherein the maximum rotation between the stops is a maximum of 150°, preferably a maximum of 120°, particularly preferably a maximum of 100° and/or wherein the switching states for the switching valve (2) are inhalation, exhalation, and pause, wherein for the inhalation the position of the valve body (23) enables a flow of gas from the environment to the suction side of the blower and at the same time enables a flow of gas from the pressure side (12) via the connection (27) to the connection (35) of the second valve (3).

10. The ventilator according to at least one of the preceding claims, **characterized in that** the switching states for the switching valve (2) are inhalation, exhalation, and pause, wherein for the pause the position of the valve body (23) enables a flow of gas from the suction side (11) of the blower (10) to the pressure side (12), as a result of which the flow of gas in at least one of the two directions from the environment (15) to the patient (14) or from the patient to the environment is substantially prevented.

11. The ventilator (1) according to at least one of the preceding claims, **characterized in that** at least one stop (32) is formed on the valve housing (39).

12. The ventilator (1) according to at least one of the preceding claims, **characterized in that** the rotary valve body (33) has openings (63, 64, 65) which face in the radial direction (61) and axial direction (62), wherein
the opening (63) to the ambient air is arranged axially in the cover disk (37) of the rotary valve body and a rotation of the rotary valve body brings the opening (63) into overlap with the connection (36) to the ambient air.

13. The ventilator (1) according to at least one of the preceding claims, **characterized in that** the openings (64, 65) are arranged radially in the rotary valve body and, in the corresponding switching position of the rotary valve body, enable a flow of gas from the pressure side (12) via the connection (27) of the switching valve (2) to the connection (35) of the second valve (3) and further via the connection (34) to the patient line (14).

## Revendications

1. Appareil respiratoire (1) comprenant une source de gaz (10), au moins une conduite de gaz (9, 11, 12, 13) et une conduite de patient (14) et au moins deux valves (2, 3, 50), dans lequel chacune des valves présente au moins indirectement un raccord vers l'air ambiant (15, 16) et dans lequel les valves sont disposées dans la conduite de gaz ou font partie de la conduite de gaz, dans lequel les valves (2, 3) sont reliées à la source de gaz (10) et à la conduite de patient (14) ou à l'air ambiant (15, 16) au moins temporairement de façon à transporter du gaz, dans lequel l'une des valves est une valve de commutation (2), dans lequel l'autre valve (3) est une valve oscillatoire et agit de telle façon que la résistance à l'écoulement dans la conduite de gaz peut être modifiée entre le ventilateur et le patient par ouverture et fermeture progressive de la valve, moyennant quoi, pendant l'insufflation et/ou l'exsufflation, des oscillations de flux et de pression sont produites, dans lequel la valve oscillatoire (3) présente au moins une ouverture menant à la conduite de patient et une autre ouverture menant à l'air ambiant et dans lequel la valve (3) est conçue de manière à fermer ou ouvrir au moins partiellement l'ouverture menant à la conduite de patient ou l'ouverture menant à l'air ambiant pour un flux de gaz respiratoire, en fonction de la position de valve, dans lequel la valve oscillatoire (3) est réalisée comme une valve rotative comprenant un corps de valve rotative (33), un moteur (31) et des ouvertures (63, 64, 65) dans plusieurs plans dans un boîtier de valve (39), dans lequel, dans un état de commutation de pause, une rotation du corps de valve rotative (33) libère l'ouverture (63) avec une ouverture vers l'environnement (36) et permet un écoulement de gaz ou une dépressurisation vers l'environnement (16) et la conduite de patient (14) est raccordée directement à l'environnement en contournant la valve de commutation (2).

2. Appareil respiratoire (1) selon l'une au moins des revendications précédentes, **caractérisé en ce que** l'une au moins des valves (2, 3) est conçue comme une valve montée de façon rotative et/ou en que l'une au moins des valves (2, 3) est conçue comme une valve axiale.

3. Appareil respiratoire (1) selon l'une au moins des revendications précédentes, **caractérisé en ce que** la valve oscillatoire (3) présente un moteur entraîné électriquement (21) avec un stator et un corps de valve (33) fixé au rotor de façon solidaire en rotation, dans lequel le corps de valve (33) est monté de façon rotative autour d'un axe de rotation (60) et une direction radiale et une direction axiale (61, 62) du corps de valve (33) sont définies sur la base de l'axe de rotation (60).

4. Appareil respiratoire (1) selon l'une au moins des revendications précédentes, **caractérisé en ce que** le corps de valve (33) de la valve oscillatoire (3) présente une région (66) constituant la cavité (38) destinée à l'arbre de moteur et présente en outre une région de paroi (68) fermant ou ouvrant au moins partiellement l'ouverture menant à la conduite de patient ou l'ouverture menant à l'air ambiant, dans une position de rotation de la valve, pour un flux de gaz respiratoire, et au moins une ouverture (63, 64, 65) de la valve libère ainsi un flux de gaz respiratoire vers la conduite de patient ou un flux de gaz vers l'environnement (16), en fonction de la position de rotation de la valve.

5. Appareil respiratoire (1) selon l'une au moins des revendications précédentes, **caractérisé en ce que** la valve oscillatoire (3) est conçue comme une valve montée de façon rotative et le corps de valve (33) présente au moins une ouverture (64, 65) dans la direction radiale (61) et au moins une ouverture (63) dans la direction axiale (62), dans lequel l'ouverture (63) dans la direction axiale (62) est orientée vers le raccord (36) menant à l'air ambiant (16).

6. Appareil respiratoire (1) selon l'une au moins des revendications précédentes, **caractérisé en ce que** le corps de valve (33) de la valve oscillatoire présente une région centrale (66), laquelle s'étend en forme de cylindre dans la direction axiale (62) autour de la cavité (38) destinée à l'arbre de moteur, et le corps de valve (33) présente en outre un disque de recouvrement (37), lequel s'étend dans la direction radiale (61) à partir de l'extrémité supérieure de la région centrale (66), ainsi qu'une région de paroi (68) au niveau du bord extérieur radial ou à proximité du bord extérieur radial du disque de recouvrement (37), laquelle s'étend dans la direction axiale (62) ou sous un angle droit à partir du disque de recouvrement et essentiellement parallèlement à la région centrale (66), dans lequel un canal (69) transportant du gaz s'étend entre la région de paroi (68) et la région centrale (66) et les ouvertures (63 64, 65) sont reliées par le biais du canal de manière à transporter du gaz.

7. Appareil respiratoire (1) selon l'une au moins des revendications précédentes, **caractérisé en ce que** la forme de l'ouverture (63) et ou la forme du raccord (36) sont conçus de telle façon que la superposition entre l'ouverture (63) et le raccord (36) augmente ou diminue linéairement selon le sens de rotation lors de la rotation de la valve.

8. Appareil respiratoire selon l'une au moins des revendications précédentes, **caractérisé en ce que** l'unité de commande (4) est configurée et réalisée pour commander le ventilateur (10) et/ou pour commander la valve de commutation (2) et/ou pour commander la valve oscillatoire (3), dans lequel, pour l'insufflation à travers le ventilateur pour un temps défini, une pression de niveau correspondant (301) est spécifiée et puis la valve de commutation (2) et/ou le ventilateur (10) commute sur l'exsufflation (305), ce pour quoi la pression (301) est abaissée à un niveau négatif correspondant dans un laps de temps défini et maintenue pour une durée déterminée, dans lequel la pression est ensuite de nouveau augmentée par le ventilateur au niveau souhaité pour la pause (306), dans lequel la pression présente une légère surpression pendant la pause (306), laquelle est en particulier comprise entre 2 et 15 mbar.

9. Appareil respiratoire (1) selon l'une au moins des revendications précédentes, **caractérisé en ce que** le moteur (21) met le corps de valve (23) en rotation entre au moins trois états de commutation, dans lequel la rotation maximale entre les butées est de 150° maximum, de préférence de 120° maximum, de façon particulièrement préférée de 100° maximum et/ou dans lequel les états de commutation pour la valve de commutation (2) sont l'inspiration, l'expiration et la pause, dans lequel, pour l'inspiration, la position du corps de valve (23) libère un flux de gaz à partir de l'environnement vers le côté aspiration du ventilateur et permet simultanément un flux de gaz à partir du côté pression (12) vers le raccord (35) de la deuxième valve (3) par le biais du raccord (27).

10. Appareil respiratoire selon l'une au moins des revendications précédentes, **caractérisé en ce que** les états de commutation pour la valve de commutation (2) sont l'inspiration, l'expiration et la pause, dans lequel, pour la pause, la position du corps de valve (23) libère un flux de gaz à partir du côté aspiration (11) du ventilateur (10) vers le côté pression (12), moyennant quoi le flux de gaz dans l'une au moins des deux direction à partir de l'environnement (15) vers le patient (14) ou à partir du patient vers l'environnement est quasiment empêché.

11. Appareil respiratoire (1) selon l'une au moins des revendications précédentes, **caractérisé en ce qu'**au moins une butée (32) est réalisée sur le boîtier de valve (39).

12. Appareil respiratoire (1) selon l'une au moins des revendications précédentes, **caractérisé en ce que** le corps de valve rotative (33) présente des ouvertures (63, 64, 65) orientées dans la direction radiale (61) et la direction axiale (62), dans lequel l'ouverture (63) vers l'air ambiant est disposée axialement dans le disque de recouvrement (37) du corps de valve rotative et une rotation du corps de valve rotative amène l'ouverture (63) en recouvrement avec le raccord (36) vers l'air ambiant.

13. Appareil respiratoire (1) selon l'une au moins des revendications précédentes, **caractérisé en ce que** les ouvertures (64, 65) sont disposées radialement dans le corps de valve rotative et, dans la position de commutation correspondante du corps de valve rotative, permettent un flux de gaz à partir du côté pression (12) vers le raccord (35) de la deuxième valve (3) par le biais du raccord (27) de la valve de commutation (2) et vers la conduite de patient (14) par le biais du raccord (34).
